(19) Europäisches Patentamt
European Patent Office
Office européen des brevets

(11) Publication number: **0 110 089**
**B1**

(12) # EUROPEAN PATENT SPECIFICATION

(45) Date of publication of patent specification: **07.01.88**

(51) Int. Cl.$^4$: **C 08 F 8/40**, C 07 C 121/30

(21) Application number: **83110337.9**

(22) Date of filing: **17.10.83**

(54) **Polymer-bound alkyl diarylphosphinite catalyst compositions and processes for making same and using same for selective conversion of acrylonitrile into 1,4-dicyano-1-butene.**

(30) Priority: **03.11.82 US 438686**
**03.11.82 US 438687**

(43) Date of publication of application:
**13.06.84 Bulletin 84/24**

(45) Publication of the grant of the patent:
**07.01.88 Bulletin 88/01**

(84) Designated Contracting States:
**DE FR GB IT NL**

(56) References cited:
**EP-A-0 010 886**
**EP-A-2 027 695**
**US-A-3 708 462**

(73) Proprietor: **ALLIED CORPORATION**
**Columbia Road and Park Avenue**
**P.O. Box 2245R (Law Dept.)**
**Morristown New Jersey 07960 (US)**

(72) Inventor: **Boyle, William Johnston, Jr.**
**15 Indian Rock Road**
**Warren New Jersey 07060 (US)**
Inventor: **Mares, Frank**
**32 Valley Forge Drive**
**Whippany New Jersey 07981 (US)**
Inventor: **Wallo, Andrea Mary**
**434 Sussex Avenue**
**Morristown New Jersey 07960 (US)**

(74) Representative: **Myerscough, Philip Boyd et al**
**J.A.Kemp & Co. 14, South Square Gray's Inn**
**London, WC1R 5EU (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to polymer-bound alkyl diarylphosphinite catalysts of formula (I):

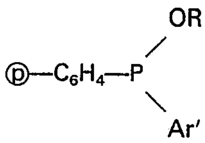

(I)

wherein the trivalent phosphorus atom is substituted by one (cyclo) alkoxy group and one aryl group and wherein the third bond of phosphorus is a P—C bond to a pendant aryl group of a polymer matrix, processes for making same and their use as a catalyst in the selective conversion of acrylonitrile into 1,4-dicyano-1-butene.

The dimerization of acrylonitrile to 1,4-dicyano-1-butene has been much investigated as a route to adiponitrile which is hydrogenated to hexamethylene diamine, a nylon 6,6 monomer.

A process for the dimerization of acrylonitrile in the presence of phosphines ($PR_3$) and phosphites ($P(OR)_3$) to give a 2:1 mixture of 2,4-dicyano-1-butene and cis- and trans-1,4-dicyano-1-butenes is disclosed in C.A., Vol. 62 (1965), 14508e (D. W. Henberg, et al.).

Tetrahedron Letters (1966) No. 51, pp. 6347—51 (W. H. Dietsche) discloses that alkyl diarylphosphinites having the formula $Ar_2POR$ in the presence of t-butanol or aqueous acetic acid effects dimerization of acrylonitrile to 2,4-dicyano-1-butene (2-methylene-glutaronitrile) and 1,4-dicyano-1-butene. The dimerization of acrylonitrile (ACN) in the presence of various trivalent oxygen-containing phosphorus (III) catalyst compositions and a mixture of a hydrocarbon such as toluene and a proton-donating solvent such as 2-propanol has been disclosed in a series of U.S. patents granted to personnel of Imperial Chemical Industries (ICI).

U.S. Patent No. 4,102,915 (Jennings et al.) discloses that a process for dimerization of ACN to substantially linear $C_6$ dimers using soluble organic phosphinites or phosphonites as catalysts is effected in the presence of a proton-donating solvent and optionally a hydrocarbon co-solvent, wherein ACN and solvents are dry and free of oxygen and wherein at least one of the solvents has a boiling point higher than ACN and is capable of phase separation with respect to dimeric products, to enable unreacted ACN to be removed by distillation and the solvent(s) and dimeric products to be separated.

U.S. Patent No. 4,316,857 (Gilbert) discloses a soluble phosphonite or phosphinite catalyzed ACN dimerization process that uses as a solvent a mixture of a proton-donating organic solvent, an aromatic hydrocarbon solvent and an aliphatic hydrocarbon solvent in a specified ratio so as to facilitate product isolation such as by phase separation or liquid extraction.

U.S. Patent No. 4,238,422 (Cozens et al.) discloses soluble aryl phosphinites and phosphonites useful as ACN dimerization catalysts wherein the aryl groups are substituted by at least one electron-donating group.

U.S. Patent Nos. 4,138,428 and 4,190,616 (Jennings et al.) disclose ACN dimerization process and soluble organic phosphinite or phosphonite catalysts having at least one aryl group substituted by electron-donating substituents.

U.S. Patent No. 4,263,224 (Jennings et al.) discloses ACN dimerization process wherein an aryl phosphonite or phosphinite is added as a low-cost scavenging reagent to a mixture of ACN and organic solvent to remove therefrom residual traces of water or other catalyst deactivating impurities before contacting said reaction mixture with a more expensive soluble aryl phosphinite or aryl phosphonite dimerization catalyst.

U.S. Patent No. 4,126,632 (Hogan et al.) discloses a process for the dimerization of ACN to straight chain 1,4-dicyanobutenes by contacting ACN with organic phosphinite or phosphonite catalyst having the formula $R_1R_2R_3P$ or $(R_1R_2P)_2R_4$ wherein at least one of the R groups $R_2$ or $R_3$ is alkoxy or cycloalkoxy and $R_4$ is alkylene or alkylenedioxy in the presence of an inert proton-donating solvent and optionally an inert hydrocarbon co-solvent. While said Hogan et al. patent also provides examples of soluble phosphinites and phosphonite wherein groups $R_1$ to $R_4$ are alkyl, aryl, cycloalkyl, polyalkylene, etc., the patent also discloses without examples that groups $R_1$ to $R_4$ may also be part of a polymeric backbone, for example, polystyrene or polyvinylalcohol or be linked to an inorganic support, for example, silica or alumina, so as to form a heterogeneous catalyst.

U.S. Patent Nos. 4,059,542 and 4,089,890 (Jennings, et al.) disclose that silica- or alumina-bound phosphinites or phosphonites as heterogeneous, i.e., insoluble catalyst compositions may only be used for the gas-phase dimerization of acrylonitrile at temperatures above 150°C. When the best phosphinite-bound to silica catalyst disclosed in U.S. Patent Nos. 4,059,542 and 4,089,890 was employed for dimerization of ACN in the gas phase at 170—190°C, only low conversions (7—20%) of ACN into an economically unattractive 3:1 (maximum value) mixture of straight and branched chain dimers and an unspecified amount of oligomers were observed.

The processes using soluble catalysts disclosed in these ICI patents produce 1,4-dicyano-1-butene, the desired linear dimer, at moderate conversions, in high selectivity with lesser amounts of the branched

dimer, methyleneglutaronitrile and oligomers. However, the ACN dimerization processes employing homogeneous, i.e., soluble alkyl diarylphosphinites substituted by electron-donating groups have the following disadvantages. At the end of each ACN dimerization run, before distillation of the desired dimeric products, the soluble phosphinite catalyst must either be removed by complicated extraction procedures or decomposed with water. The extraction procedures inherently result in appreciable losses of the soluble phosphinite catalysts for two reasons. Firstly, the differences in the solubility of the soluble phosphinite catalyst in the solvents are not infinite, and thus, several extractions of the catalyst are required. Secondly, extraction enhances the chances for contamination of the solvents, unreacted ACN and catalyst with moisture and oxygen, impurities which deactivate the catalyst. Decomposition of the soluble phosphinite catalyst by the addition of water substantially increases catalyst consumption and contaminates the reaction solvents (isopropanol and toluene) and unreacted ACN with water and/or oxygen. Thus, after extration and decomposition procedures, the reaction solvents and unreacted ACN must be degassed and redried before the recycle of same. In the case of isopropanol and ACN, degassing and redrying are very costly and time consuming steps.

One way in which the workup of the dimerization reaction could be greatly simplified, while simultaneously conserving the expensive catalyst, would be to support the catalyst on a polymer matrix. C. U. Pitman, et al. (CHEMTECH, Sept. 1973, pp. 560—566) disclose that soluble catalysts, e.g., transition metal catalyst, may be bound to polymer backbones. See also Paper No. 29 by W. O. Haag, et al., in "Proc. 5th International Congress on Catalysis", Vol. 1, pp. 465—472 (1973) and an article by D. D. Whitehurst in CHEMTECH, January, 1980, pp. 44—49.

During the course of development of the present invention, phosphinites bound to organic polymer matrices via P—O—C bonds were prepared and were found to be impractical and inactive catalysts for ACN dimerization. Similarly, phosphinites bound via P—O—M bonds to inorganic matrices (M), as disclosed in U.S. Patent Nos. 4,089,980 and 4,059,542 (ICI), possess a low activity and low selectivity and may be used only for gas phase ACN dimerizations. Although the above-identified ICI U.S. Patents mention the use of polystyrene-bound phosphinites, no working example of the use and/or preparation of same is given in the above-identified ICI U.S. Patents or in other published literature of which we are aware.

According to the present invention, we provide a polymer-bound alkyl diarylphosphinite catalyst which is substantially free of C=O, OH, NH or SH groups and is of the formula (I):

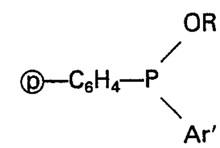

(I)

wherein ⓟ—$C_6H_4$— is bonded to the trivalent phosphorus atom through a P—C bond and is a pendant aryl group of a polymer matrix derived from a polymer ⓟ—$C_6H_5$ containing aryl groups, at least 1% of the pendant aryl groups of the polymer matrix being bound to phosphorus, Ar' is an aromatic group of the formula:

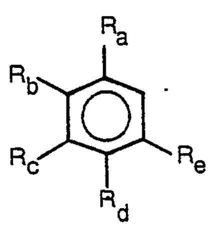

wherein $R_a$, $R_b$, $R_c$, $R_d$ and $R_e$ are independently heteroatom-containing electron-donating groups selected from —$OR^3$ and —$N(R^4, R^5)$, or hydrogen, alkyl of 1 to 10 carbon atoms or cycloalkyl of 5 to 10 carbon atoms, or two of said $R_a$ to $R_e$ groups from part of a fused alicyclic ring and the remainder of said $R_a$ to $R_e$ groups are as hereinbefore defined, and R, $R^3$, $R^4$ and $R^5$ are independently straight or branched chain alkyl of 1 to 10 carbons or cycloalkyl of 5 to 10 carbon atoms.

The present invention also provides a process for preparation of such a catalyst which comprises the steps of:

(a) brominating at least 1%, preferably 5 to 100%, of the pendant aromatic groups of a polymer matrix of the formula ⓟ—$C_6H_5$;

(b) contacting the brominated aromatic-containing polymer produced in step (a) with at least a stoichiometric amount, e.g. 2—3 equivalents, of a lithium compound selected from $LiNH_2$, $LiNHR^7$, $LiNHR^7R^8$ and $LiR^7$ wherein $R^7$ and $R^8$ are independently alkyl of 1 to 8 carbons or cycloalkyl groups of 5 to 12 carbons;

(c) contacting the lithiated aromatic-containing polymer produced in step (b) with, e.g. an excess 2 to 3 equivalents, an aryl phosphorus compound selected from Ar'P(X)OR, $Ar'P(OR)_2$ and $Ar'PX_2$ wherein Ar' and R are as hereinbefore defined and X is halogen (preferably chlorine);

(d) recovering a polymeric substance having the formula ⓟ—$C_6H_4$—P(Ar')OR or ⓟ—$C_6H_4$—P(Ar')X when Ar'$P(X)_2$ is used in step (c); and

(e) when ⓟ—$C_6H_4$—P(Ar')X is formed in step (d) converting it into ⓟ—$C_6H_4$—P(Ar')OR by reaction with ROH and pyridine.

The present invention further provides a process for the preparation of a catalyst as defined above which process comprises the steps of:

(a) contacting a polymer ⓟ—$C_6H_5$ with $PX_3$, in the presence of an effective amount of a Friedel-Crafts catalyst (preferably selected from $TiX_4$, $ZrX_4$, $BX_3$, $SbX_5$, $SnX_4$ and $AlX_3$ where X is halogen, preferably chlorine, especially $PCl_3$ or $AlCl_3$), for a time sufficient to produce a polymer wherein at least 1%, preferably 5 to 100%, of the pendant aromatic groups are of the formula —$C_6H_4$—$PX_2$ wherein X is F, Cl, Br or I;

(b) contacting the polymer produced in step (a), in the presence of an effective amount of a Friedel-Crafts catalyst, with an aromatic compound Ar'H wherein Ar' is as defined above for a time sufficient to produce a polymer having formula ⓟ—$C_6H_4$—P(Ar')X; and

(c) contacting the product of step (b) with an alkanol or cycloalkanol of the formula ROH, wherein R is as defined above (preferably ethanol or 2-propanol), in the presence of a base (preferably pyridine) for a time sufficient to produce a polymer of formula ⓟ—$C_6H_4$—P(Ar')OR.

The present invention also contemplates the specific catalysts of the following formulas:

ⓟ—$C_6H_4$—P[p-$CH_3OC_6H_4$—]OR; ⓟ—$C_6H_4$—P[$(CH_3)_3C_6H_2$—]—OR

and

ⓟ—$C_6H_4$—P[$(CH_3)_3C_6H_2$—]—Cl,

wherein ⓟ—$C_6H_4$ is derived from the polymer polystyrene (ⓟ—$C_6H_5$) and wherein R is cyclohexyl, $CH_3$—, $C_2H_5$— or i-$C_3H_7$.

The present invention also provides a process for conversion of acrylonitrile into 1,4-dicyano-1-butene which comprises contacting a liquid phase comprising acrylonitrile with an effective amount of a polymer-bound alkyl diarylphosphinite catalyst of formula (I) above for a time sufficient to effect conversion to a stream containing 1,4-dicyano-1-butene.

The invention will be described more fully below by reference to the drawings in which:

Figures 1a and 1b are $^{31}P$ NMR Spectra of preferred embodiments of a polymer-bound phosphinite catalyst of the present invention.

Figure 2 schematically illustrates the use of the polymer-bound phosphinite catalyst of the present invention in a flow reactor.

Figure 3 graphically illustrates the variation in the percent conversion of ACN vs reaction time for the polymer-bound phosphinite catalyst of the present invention used as shown in Figure 2.

Figure 4 schematically illustrates one synthetic pathway for preparation of a polymer-bound alkyl diarylphosphinite catalyst of the present invention.

Figure 5 schematically illustrates another synthetic pathway for preparation of a polymer-bound alkyl diarylphosphinite catalyst of the present invention.

Figure 6 schematically illustrates the reaction of a polymer-bound dialkyl arylphosphonite with water to form a product that does not react with ACN.

Figure 7 schematically illustrates the reaction of polymer-bound dialkyl arylphosphonite with oxygen to form catalytically inactive phosphorus [V].

Figure 8 schematically illustrates a method of preparation of a polymer-bound dialkyl phosphonite.

The present invention provides polymer-bound alkyl diarylphosphinite catalysts of formula (I) useful as catalysts in the dimerization of acrylonitrile. The dimerization may be operated batchwise or continuously with high selectivity and high percent conversion by contacting a liquid phase containing acrylonitrile with a polymer-bound alkyl diarylphosphinite catalyst of formula (I), positioned in a fixed bed or in a flow reactor, for a time sufficient to effect conversion of ACN to a stream containing 1,4-dicyano-1-butene. Thus, the catalyst compositions of the present invention afford batchwise or continuous operation of the dimerization of ACN, facile product separation and recovery of same. In addition, the reaction medium of unreacted acrylonitrile and at least one inert and a proton-donating solvent may be recycled for further ACN dimerization while maintaining high selectivity to the preferred linear dimer, 1,4-dicyano-1-butene, with high percent conversion of ACN. Further, the immobilization of the phosphinite catalyst on a polymer matrix eliminates the need for the complicated workup procedures of the prior art and minimizes degradation of expensive active polymer-bound catalysts by exposure of same to water and air. In batchwise operation, the polymer-bound phosphinite catalyst is separated by a simple filtration; in continuous operation, no separation is necessary because the effluent from the flow reactor is free of catalyst. In either case, linear and branched chain dimers and oligomers of acrylonitrile are conveniently and easily separated from the unreacted acrylonitrile, at least one inert hydrocarbon solvent and the proton-donating solvent by a simple distillation. The condensed volatile components — unreacted acrylonitrile, at least one inert hydrocarbon solvent, and a proton-donating solvent — can be recycled to a batch or continuous reactor without any further purification because contamination of the condensed volatile components by water or oxygen has been practically eliminated.

The polymer-bound alkyl diarylphosphinite of the present invention has demonstrated long-term stability. For example when the dimerization of acrylonitrile was effected continuously over 184 hours using a flow reactor packed with a preferred embodiment of the polymer-bound catalyst of formula (I) wherein R is ethyl or isopropyl and Ar' is Ⓟ—$CH_3OC_6H_4$—, i.e. with Ⓟ—$C_6H_4$—P—(p-$CH_3OC_6H_4$—)O—$C_2H_5$ or Ⓟ—$C_6H_4$—P(p-$CH_3OC_6H_4$—)O—i-$C_3H_7$, the percent conversion slowly declined from 63% initially to 40% at 184 hours. The percent selectivity to linear and branched dimers was 90—91% (92% linear, 8% branched). In contradistinction, trimethylsilyl dibutyl phosphinite, a soluble phosphinite having a P—O—Si bond and serving as a model compound for the phosphinites bound to silica such as disclosed in U.S. Patent Nos. 4,059,542 and 4,089,890, was prepared (see Example 12) and was tested as an ACN dimerization catalyst but was found completely inactive and was, in a relatively short time (3 hours), in the ACN dimerization medium, converted into a catalytically inactive phosphorus (V) species. Partially phosphinited derivatives of polyvinyl alcohol and of various hydroxyl-containing polymers were prepared and showed little or no activity as ACN dimerization catalysts, in agreement with the results of other studies conducted with 1,2- and 1,3-diols, which are not reported in the experimental section hereinbelow (see Comparative Example 22 and Table V). Fully phosphinited derivatives of hydroxyl-containing polymers required addition of proton donors, e.g., isopropanol or neopentyl alcohol, to the ACN dimerization medium. The presence of such proton donors caused solvolysis of the polymer-bound phosphinites and the formation of soluble monomeric phosphinites (see Table V for a summary of these results).

Phosphinite bound to polyvinyl alcohol (PVA), PVA cross-linked with TDI and similar OH-containing polymers such as 4-(2-hydroxypropyl)polystyrene and "TOYOPEARL", a trade name for a cross-linked PVA copolymer containing aliphatic hydroxy and non-hydroxyl oxygen moieties were found to be impractical or ineffective catalysts for ACN dimerization.

Polymer-bound di(cyclo)alkyl arylphosphonites have been found to be useful for lowering the water content of the liquid phase comprising acrylonitrile, inert solvent and proton-donating solvent and containing less than 30 ppm of water, preferably less than 15 ppm to a value less than 5 ppm or even 2—3 ppm of water.

The polymer-bound di(cyclo)alkyl arylphosphonite is conveniently contained in a fixed bed, i.e. a column positioned before the polymer-bound phosphinite catalyst as illustrated in Figure 2. The polymer-bound di(cyclo)alkyl arylphosphonite may be prepared by the scheme illustrated in Figure 8 and recycled, i.e. reconverted to phosphonite by the scheme illustrated in Figure 6. The (cyclo)alkyl groups in the polymer-bound di(cyclo)alkyl arylphosphonite are acyclic groups having 1 to 10 carbons and cyclic groups having 5 to 10 carbons. Secondary aliphatic groups of 3 to 10 carbons and cyclic groups having 5 to 12 carbons are more preferred. Isopropyl groups are most preferred. Primary alkyl groups are less preferred. Tertiary alkyl and aryl groups, e.g. tert-butyl and phenyl, are to be avoided.

By the term "% conversion", as used herein, is meant the % by weight of acrylonitrile (ACN) that is converted to total linear/branched dimeric, oligomeric and polymeric products.

By the term "% selectivity", as used herein, is meant the % by moles of acrylonitrile converted into linear dimers, i.e. cis- and trans-1,4-dicyano-1-butene (DCB—1). Thus, % selectivity is defined as twice the number of moles of DCB—1 divided by moles of reacted acrylonitrile multiplied by 100%.

The process for dimerization of acrylonitrile using the polymer-bound alkyl diarylphosphinite catalyst compositions of the present invention is now more fully described. The liquid phase comprising acrylonitrile that is contacted with the polymer-bound alkyl diarylphosphinite further comprises at least one inert solvent and a proton-donating compound. In a preferred embodiment of the process of the present invention, the liquid phase comprising acrylonitrile, at least one inert solvent and a proton-donating solvent and containing less than 30 ppm, preferably less than 15 ppm water, is contacted prior to contact with the polymer-bound phosphinite catalyst, with a recyclable polymer-bound di(cyclo)alkyl arylphosphonite which lowers the water content to 5 ppm or less. The polymer-bound phosphonite is conveniently contained in a fixed bed, i.e. a column such as illustrated in Figure 2, and may be recycled, i.e. reconverted to phosphonite by scheme illustrated in Figure 6.

The concentration (% by volume) of acrylonitrile in the liquid phase is not critical and is conveniently adjusted between about 5% and 50% to control the rate of reaction. Concentrations of acrylonitrile above 50% in liquid phase lead to oligomer and polymer formation and should be avoided. When the concentration of acrylonitrile is below 5% in liquid phase, the reaction rate is too low to be practical.

Inert solvents are those compounds which do not react or interfere with the process or catalyst or reactants of the present invention. The inert solvents found useful in the present invention are liquid, non-hydroxylic aromatic hydrocarbon solvents, including hydrocarbons such as benzene, xylenes, toluene, and polyalkylbenzenes. Toluene is the preferred inert solvent solely for economic reasons.

Mixtures of liquid aromatic hydrocarbons and different amounts of liquid aliphatic hydrocarbons may be used without interfering with the process of the present invention so long as the liquid phase remains homogeneous. Thus, aliphatic or alicyclic hydrocarbons such as petroleum ether or cyclohexane may be used with sufficient amounts of aromatic hydrocarbon(s), preferably toluene, to insure the solubility of the proton-donating solvents. Solvents such as ethers and nitriles may also be used. However, the selectivity to the desired linear dimer is decreased when the liquid phase contains polar solvents, and use of such solvents is to be avoided.

Proton-donating solvents such as aliphatic or cyclic alcohols are added to facilitate the selectivity of the

dimerization process of the present invention. Proton-donating solvents of higher acidity than alcohols such as organic carboxylic or sulfonic acids, as well as thiols and phenols, interfere with the catalyst and must be avoided.

Secondary aliphatic alcohols having 3—10 carbons and cyclic secondary alcohols having 5 to 10 carbon atoms are preferred. Primary alcohols are found to effect dimerization of ACN, but the % selectivity is lower compared to the process employing secondary alcohols. Use of primary alcohols is less preferred. Tertiary alcohols interact with the polymer-bound phosphinite catalyst to form t-alkyl phosphinites which eliminate olefins leaving behind catalytically inactive secondary phosphine oxides. Thus, the use of tertiary alcohols is to be avoided.

Since a fixed bed or flow system, preferably a flow system, is employed, only the ratio of proton-donating solvent, preferably isopropanol:inert solvent, preferably toluene:acrylonitrile can be specified. The ratio (volume) of proton-donating solvent to inert solvent to acrylonitrile in the liquid phase is 0.2—10:10—0:1—7.5. In the preferred embodiment of the present invention, the ratio (volume) of isopropanol to toluene to acrylonitrile is 1:10:3. A portion of toluene may be conveniently replaced by cyclohexane and hexamethylbenzene (internal standards).

The contact time is sufficient to convert 5—95%, preferably 30—80% of the acrylonitrile, into the desired dimeric product. Contact times may conveniently be from 10 minutes to several hours.

The presence of water and oxygen interferes with the process or catalyst of the present invention and must be avoided. Thus, acrylonitrile, the inert and proton-donating solvents and reactor must be rigorously dried before use. The contacting of ACN with the catalyst of the present invention is performed under substantially anhydrous conditions, i.e. the concentration of water in liquid phase is preferably maintained less than 30 ppm, and more preferably less than 15 ppm, and most preferably less than 5 ppm. Conventional drying agents, such as $CaH_2$, 3A or 4A molecular sieves, and polymer-bound di(cyclo)alkyl arylphosphonites may be employed to dry the liquid phase before the start of the reaction to a value less than 5 ppm. The dimerization process of the present invention should be operated under a dry inert atmosphere, e.g. nitrogen gas, to avoid contamination by oxygen or water vapor during the operation of the process of the present invention. Stabilizers normally present in acrylonitrile such as hydroquinones or phenol-type compounds are to be removed before contacting with the catalyst in the process of the present invention. In addition, scavenging agents, such as aryl phosphonites that are disclosed in U.S. Patent No. 4,263,224, may be conveniently added to liquid phase reservoirs prior to contacting same with polymer-bound alkyl diarylphosphinite catalyst of the present invention. Concentrations of less than one % by volume in the liquid phase of the scavenger agents are normally sufficient to remove chemicals such as $H_2O$ that would adversely affect the polymer-bound catalyst of the present invention. In a preferred embodiment of the present invention, polymer-bound di(cyclo)alkyl arylphosphonites having the formula Ⓟ—$C_6H_4$—$P(OR)_2$ wherein R is an alkyl group of 1 to 10 carbons or cycloalkyl group of 5 to 10 carbons, preferably a secondary alkyl group such as isopropyl, are used to lower the water content of the liquid phase that contains acrylonitrile, inert solvent, and proton-donating solvent, and that has been subjected to conventional drying by reagents such as $CaH_2$ and molecular sieves, to a value of less than 15 ppm, preferably 5 ppm or lower, i.e. 2—3 ppm. The preparation of the polymer-bound di(cyclo)alkyl arylphosphonites is shown schematically in Figure 8. See Example 7 hereinbelow. Levels of water in excess of 50 ppm in the liquid phase may be tolerated. However, lower selectivities and deactivation of large amounts of catalyst are to be expected.

It is a special feature of the process of the present invention that the drying agent comprising polymer-bound dialkyl phosphonite reacts with water to form a polymer-bound alkyl phosphinite having the formula Ⓟ—$C_6H_4$—P=O(OR)(H) that does not react with ACN but that may be conveniently reconverted into polymer-bound dialkyl arylphosphonite by a sequence of steps outlined in Figure 6. It is another special feature of the process of the present invention that the sequence of steps outlined in Figure 6 may be performed without removing same from the container used to dry the ACN dimerization reaction medium. The polymer-bound phosphinate having the formula Ⓟ—$C_6H_4$—P=O(OR)(H) may conveniently be treated with a reagent such as $PX_3$, $COX_2$, wherein X is halogen, preferably $PCl_3$, for a time sufficient to produce a polymer-bound alkyl aryl-phosphonous dichloride having the formula Ⓟ—$C_6H_4$—$P(Cl)_2$. The polymer-bound phosphonous dichloride is then treated with a primary or secondary alcohol in the presence of a base, such as pyridine, for a time sufficient to produce a polymer-bound dialkyl arylphosphonite. The preferred alkanol is isopropanol. The preferred polymer is polystyrene cross-linked with at least 1 to 40 weight percent divinylbenzene and having the form of micro- or macro-reticular beads or clusters of beads.

The reaction temperature for ACN dimerizations of the present invention is commonly in the range of 0°C to 100°C. Temperatures in the range of 50°C to about 75°C are preferred. A temperature of 60°C is more preferred.

Reaction pressure is not critical and may be sub- or super-atmospheric as well as atmospheric. When the polymer-bound catalyst operates in a flow reactor, at temperatures above 50°C, super-atmospheric pressures are commonly in the range of 1—5 atm, and preferably 1.5—3 atm.

The polymer-bound catalyst of the present reaction comprises a polymer-bound alkyl diarylphosphinite having the formula (I), Ⓟ—$C_6H_4$—P(Ar')OR wherein the trivalent phosphorus is substituted by

one alkoxy group and one aryl group and wherein the third bond of phosphorus is a P—C bond to a pendant aryl group of a polymer matrix.

Among the polymers of the polymer-bound catalyst found useful in the present invention are polystyrene and polystyrene cross-linked with 1 to 40 percent by weight of divinylbenzene and in the form of micro- or macro-reticular beads or clusters of beads. Preferably, the polymer of the polymer-bound catalyst comprises polystyrene cross-linked with 1 percent of divinylbenzene.

By the term "effective amount of polymer-bound catalyst", as used herein, is meant that at least 1% of the pendant aryl groups of polystyrene in formula (I) is substituted with phosphorus in the form of phosphinite. Conveniently, at least 5 to 100% of the pendant aromatic rings bound to polymer backbone are substituted by phosphorus. In the preferred mode of preparation of polymer-bound catalyst of the present invention, 25—100%, preferably 80% or more, of the phosphorus bound to the aromatic rings was in the form of phosphinite.

The aromatic ring (Ar′) bound only to phosphorus in the polymer-bound alkyl diarylphosphinite is as defined above. Preferably at least one of the $R_a$ to $R_e$ groups is a heteroatom-containing electron-donating group selected from alkoxy and N,N-dialkylamino. Aromatic rings containing at least one heteroatom-containing electron-donating group or three alkyl groups are preferred. Aromatic rings containing only H are less preferred.

Among the heteroatom-containing electron-donating groups found useful in the catalyst of the present invention are (cyclo)alkoxy ($OR^3$), N,N-dialkylamino [—N($R^4$,$R^5$)] wherein the (cyclo)alkyl groups $R^3$, $R^4$, $R^5$ are straight or branched chain aliphatic groups of one to ten carbons or cycloalkyl groups of five to ten carbons. Preferred heteroatom-containing electron-donating groups are $CH_3O$—, $C_2H_5O$—, i-$C_3H_7O$—, $(CH_3)_2N$—, $(C_2H_5)_2N$—, and (n-$C_3H_7)_2N$.

Among the aromatic groups attached only to phosphorus that are useful in the polymer-bound diaryl phosphinite catalyst of the present invention are p-alkoxylphenyl, p-N,N-dialkylaminophenyl, 2,3,4,5-tetra-alkylphenyl; 3,4,5-, 2,3,5-, 2,4,5- and 2,3,4-trialkylphenyl wherein alkyl has one to ten carbons and is preferably methyl.

The polymer-bound alkyl diarylphosphinite catalysts of the present invention contain at least 1% of aromatic groups pendant to the polymer matrix and bound to phosphorus and are substantially free of C=O groups such as e.g., aldehydes, ketones, esters and amides, or —OH, NHR or —$NH_2$ or SH that may react with phosphinite phosphorus or otherwise interfere with activity of the phosphinite as a selective acrylonitrile dimerization catalyst. Preferred polymer-bound catalysts of the present invention include Ⓟ—$C_6H_4$—P(p-$CH_3OC_6H_4$—)OR, Ⓟ—$C_6H_4$—P$(CH_3)_3C_6H_2$—]OR; wherein Ⓟ—$C_6H_4$— is derived from a polymer which comprises polystyrene (Ⓟ—$C_6H_5$), preferably which consists essentially of polystyrene cross-linked with at least 1 weight % of divinylbenzene and more preferably polystyrene cross-linked with 1—40 weight % of divinylbenzene and in the form of micro- or macro-reticular beads or clusters of beads; and wherein R is a straight or branched chain aliphatic group having one to ten carbon atoms and cycloalkyl groups having five to ten carbon atoms. Preferred R groups are cyclohexyl, methyl, ethyl and groups having formula $R^1R^2C(H)$— wherein $R^1$ and $R^2$ are independently selected from hydrogen and straight and branched chain alkyl groups having 1 to 9 carbon atoms such as isopropyl.

Figure 5 schematically illustrates a preferred synthetic pathway for preparation of polystyrene-bound alkyl diarylphosphinites of the present invention (see also claim 7).

The bromination reaction illustrated in Figure 5 is conventional and results in almost exclusive parabromination and is described by S. E. Jacobson, et al., in JACS, 1979, 101, 6938.

The reaction with a lithium compound which is preferably methyl or n-butyl lithium is performed in inert hydrocarbon solvents such as toluene or heptane at 50—75°C for 3 hrs. See, for example, M. J. Farrall, et al., in J. Org. Chem., 1976, *41*, 3877.

Since lithium reacts with carbon-halogen bonds, the presence of carbon-halogen bonds and double or triple bonds (that add bromine) in the polymer matrix are to be avoided.

Of the phosphiniting agents employed, the use of the phosphonochloridous esters, Ar′PCl(OR), wherein Ar′ is substituted with heteroatom-containing electron donating groups such as alkoxy or N,N-dialkylamino, resulted in 50—100% of the phosphorus that was incorporated into the polymer being in the desired phosphinite form and is preferred. Solely for economic reasons, ethyl p-anisylphosphonochloridite is most preferred. Of course, other electron-donating groups in addition to para-methoxy may also be used. Phosphonites having formula Ar′P$(OR)_2$ gave polymer having large amounts of P(V) and their use is to be avoided. Phosphonous dichlorides (Ar′$PCl_2$) were found to be too reactive and produced large amounts of tertiary phosphines from reaction with two different lithiated phenyl groups. Since tertiary phosphines catalyze dimerization of acrylonitrile with predominate formation of the branched-chain dimer, methyleneglutaronitrile, the use of phosphonous dichlorides is to be avoided.

The reaction of the lithiated polymer with excess phosphiniting agent, preferably phosphonochloridous esters, was performed at 0°C or less, e.g., −78°C and the reaction mixture was allowed to warm to room temperature, with stirring. The polymeric substance formed was washed repeatedly with toluene, tetrahydrofuran (THF) and a THF-isopropanol mixture to remove all traces of lithium cations and bases which interfere with the ACN dimerization process.

In another preferred aspect of the present invention schematically illustrated in Figure 4, the polymer-bound alkyl diarylphosphinite catalyst having formula I was prepared by the process defined in claim 10.

Among the aromatic compounds having the formula Ar'H found useful in the present invention are toluene, xylenes, e.g. m-xylene, trialkylbenzenes such as 1,2,3-, 1,3,4-, and 1,2,4-trialkylbenzene wherein alkyl is a linear or branched chain group having 1 to 10 carbons and tetralin. Trimethylbenzenes are preferred. Aromatic compounds containing groups such as alkoxy that react with Friedel-Crafts catalyst are to be avoided.

By the term "effective amount of a Friedel-Crafts catalyst" is meant at least one to three times the stoichiometric amount of said catalyst required for formation of the desired product.

The yield of polymer-bound alkyl diarylphosphinite obtained in the esterification step of Figure 4 is deleteriously effected by the presence of a Friedel-Crafts catalyst absorbed in the polymer-bound phosphinous chloride recovered from the previous step. To maximize the yield of polymer-bound alkyl diarylphosphinite, it is necessary, prior to the esterification step, to effect removal of Friedel-Crafts catalyst, e.g. $AlCl_3$, from polymer-bound phosphinous chloride such as by repeated washings with polar solvents such as THF or polar solvents in combination with a base such as pyridine. To achieve full activity of polymer-bound alkyl diarylphosphinite catalyst prepared via Friedel-Crafts scheme illustrated in Figure 4, it is advisable to remove the Friedel-Crafts catalyst, e.g. $AlCl_3$, as fully as possible. Removal of the residual Friedel-Crafts catalyst by use of proton donating solvents such as alcohols that react with phosphinous chlorides is to be avoided.

During the course of development of the synthetic scheme illustrated in Figure 4, there was found an article by D. D. Whitehurst in CHEMTECH, 1980, 44, wherein a scheme is disclosed suggesting that phosphinous chloride bound to polystyrene may be formed by the reaction of polystyrne with $C_6H_4PCl_2$ catalyzed by $AlCl_3$. However, in our hands, the reaction of 3,4,5-$(CH_3)_3C_6H_2PCl_2$ with polystyrene in the presence of $AlCl_3$ at 60°C failed to produce any polystyrene-bound arylphosphinous chloride. (See Example 11a.) When the reaction of 3,4,5-$(CH_3)_3C_6H_2PCl_2$ with polystyrene was conducted in the presence of $AlCl_3$ at 100°C, there was obtained a yellow-orange gummy solid that was contacted with isopropanol in the presence of pyridine; the reaction product was shown to contain no phosphinite phosphorus by $^{31}P$ NMR analysis. (See Examples 11b—c).

The preferred polymer-bound catalyst having the general formula (I) is Ⓟ—$C_6H_4$—P[$(CH_3)_3C_6H_2$]—O—i-$C_3H_7$ wherein Ⓟ—$C_6H_4$— is derived from a polymer which comprises polystyrene (Ⓟ—$C_6H_5$) and preferably consists essentially of polystyrene crosslinked with 1—40% by weight of divinylbenzene and in the form of micro- or macroreticular beads or clusters of beads.

## GENERAL EXPERIMENTAL

Infrared spectra, as either neat films or KBr pellets, were recorded on a Perkin-Elmer 283 Spectrophotometer. Proton, $^{13}C$ and $^{31}P$ NMR were recorded on Varian T—60, FT—80A, and XL—200 instruments. Phosphorus chemical shifts are reported relative to external 85% phosphoric acid. Gas chromatographic analyses were performed on a Hewlett Packard 5710 using a 7.6 × 0.3 cm (3' × ⅛") Porapak P column and a Hewlett Packard 3352B computer to monitor the retention times and peak areas.

All dimerization runs and the preparation of the polymer-bound catalysts were carried out with the careful exclusion of oxygen and moisture. Transfer of moisture-sensitive solids was carried out in a Vacuum Atmospheres Dry Box. Volatile liquids were transferred on a vacuum line, while the nonvolatile, soluble catalysts were transferred via syringe in a stream of argon.

### Materials

All solvents were reagent grade. Toluene and cyclohexane were stored over sodium-potassium alloy under vacuum. Tetrahydrofuran (THF) was stirred with lithium aluminum hydride, then distilled into a solvent reservoir containing sodium-potassium alloy and anthracene. The THF was distilled from the solution of the blue radical anion as needed. Isopropanol, tert-butyl alcohol and acrylonitrile were refluxed over calcium hydride passing through a sieve having 0.42 mm mesh aperture (−40 mesh) for at least three hours, then distilled onto flame-dried 4A molecular sieves for storage. Neopentyl alcohol was sublimed onto flamed-dried 4A molecular sieves. After standing overnight, these reagents were sampled for water content (Karl Fisher) and found to contain <30 ppm of water. Samples were taken periodically to assure that the water content remained low, i.e., less than 50 ppm. Pyridine was also dried over calcium hydride and distilled onto molecular sieves.

"TOYOPEARL® 55", the trade name for a polyvinyl alcohol cross-linked copolymer containing aliphatic hydroxy and non-hydroxy oxygen moieties, was obtained in the form of beads as a water slurry in superfine (20—30 μm) and coarse (50—100 μm) grades from MCB, and was washed repeatedly with water and with acetone, then dried *in vacuo* at 100°C for at least 24 hrs. Polyvinyl alcohol (88% hydrolyzed, avg MW 10,000) was obtained from Aldrich. Ethylene/vinyl alcohol copolymer (40/60) type F was purchased from Kuraray Co., Ltd. Polyvinyl alcohol cross-linked with terephthalaldehyde and with tolylene diisocyanate were prepared as described by G. Manecke et al. in Makromolekulare Chem. *117*, 725 (1976) and by S. Nozakura et al. in J. Polymer Sci., A, *10*, 2767 (1972).

Ethyl and isopropyl di-p-tolylphosphinites and ethyl diphenylphosphinite were prepared by following the procedure of Coezens, et al., as disclosed in U.S. Patent No. 4,238,422. p-Anisylphosphonous dichloride and diisopropyl p-Anisylphsophonite were prepared by following the procedure of Miles and co-workers (J. Org. Chem., 1975, *40*, 343). Ethyl p-anisylphosphonochloridite and other phosphonochloridous esters

were prepared by the procedure of Steininger (Chem. Ber., 1962, *95*, 2993) for the comproportionation of the corresponding phosphonous dichloride and phosphonite. Ethyl phenylphosphinate was prepared by following the procedure described in T. L. Emmick et al., J. Am. Chem. Soc., 1968, *90*, 3459. p-Tolylphenylphosphine oxide was prepared by following the procedure of M. Grayson et al. in *Tetrahedron*, 1967, Volume 23 at 1065 et. seq.

Example 1

Preparation of Phosphinited Polystyrene.

A previously prepared sample of brominated polystyrene beads passing and respectively retained on sieves having 0.074 respectively 0.037 mm mesh aperture (Bio-Beads S—X1, 200—400 mesh) containing 17.1% bromine (27% of the rings) was used in most of the preparations (see S. E. Jacobson, et al. JACS, (1979) *101*, 6938). The polymer (3.0 g, 6.4 meq $Br^-$) was placed in an H-reactor and degassed. Into the other arm of the reactor was syringed a three-fold excess of *n*-butyl lithium in hexane under a stream of argon. This was concentrated on the vacuum line to about 3 ml, then about 20 ml of dry toluene was distilled onto the butyl lithium and about 10 ml of toluene onto the polymer. The *n*-butyl lithium solution was then filtered onto the polymer at −78°C, the mixture warmed to room temperature and then heated in a mil bath at 60°C for 3 h. The suspension was then filtered through the glass frit in the H-reactor and part of the toluene was redistilled back onto the side of the polymer. After filtering again, the toluene was poured off under a stream of argon, then fresh toluene was distilled into the arm containing the polymer. The mixture was stirred briefly, then filtered, and the toluene poured out under Argon. Fresh toluene (25 ml) was again distilled onto the polymer, the mixture cooled to 0°C, then treated via syringe with the phosphiniting agent (2—3 equivalents), warmed to room temperature, and stirred for one hour. The mixture was filterd and some of the toluene distilled back onto the polymer. This was stirred briefly and filtered again, then the toluene was poured off under Argon. The polymer was then washed repeatedly with THF and with THF/isopropanol (1/1) in order to remove all traces of base, lithium chloride and unreacted phosphorus reagent. Inadequate washing of the polymer leads to formation of Michael adducts of acrylonitrile and alcohol in the dimerization runs. A typical analysis of the phosphinited polystyrene from a preparation using p-$CH_3OC_6H_4(Cl)OEt$ as phophiniting agent was: C, 83.2; H, 7.90; P, 4.32; Br, 0.11. Calculated for complete replacement of bromine with p-$CH_3OC_6H_4P(OEt)$: C, 81.0; H, 8.09; P, 5.3. A $^{31}P$ NMR spectrum from one of the better preparations is shown in Figure 1a. The large broad beak in the 100—120 ppm region (relative to 85% $H_3PO_4$) was due to phosphinite phosphorus, by comparison to soluble reference samples. The peak at −18 ppm was due to tertiary phosphine, while the two peaks in the 20—40 ppm range were due to P(V) compounds, presumably tertiary phosphine oxide (Arbuzov rearrangement product) and perhaps phosphinate or secondary phosphine oxide. The other peaks were of unknown origin. Samples prepared using phosphonous dichlorides as the phosphiniting agent showed much larger tertiary phosphine peaks, while those prepared using phosphonites as the phosphorus reagent had very large peaks in the P(V) region.

Example 2

Dimerization Runs.

In a typical dimerization run, hexamethylbenzene, an internal standard, (800 mg, 4.93 mmol) was accurately weighted and placed in a thick-walled glass reaction vessel). To this, ca. 0.37 mmol of catalyst (monomer or polymer) listed in Table I was added and degassed on a vacuum line. Next, the following dry reagents were distilled in on a vacuum line: 1 ml of isopropanol, 3 ml of acrylonitrile, and 9 ml of toluene. Approximately 1 ml of dry cyclohexane was accurately weighted into a specially designed vessel, then transferred on the vacuum line to the reaction vessel by means of a small U-shaped tube designed to minimize the distillation path and thus facilitate accurate transfer of the internal standard. The solution was then sampled under a stream of argon so that a gas chromatogram of the starting mixture could be obtained. The reaction vessel was sealed with a high vacuum stopcock and placed in an oil bath at 60°C. At varying intervals, the reactor was removed from the bath, cooled to room temperature, and sampled under a stream of argon.

Gas chromatographic analyses were performed using the Porapak P column and a temperature program starting at 60°C, increasing at 8°C/min for 4 min, then at 32°C/min to 240°C and holding for 4 min. Reference standards were prepared using cyclohexane and hexamethylbenzene as internal standards. Cyclohexane was used to calculate acrylonitrile concentration and the hexamethylbenzene was used for measuring the concentrations of methyleneglutaronitrile and the cis- and trans-1,4-dicyano-1-butene products.

Example 3

Long-term Stability of Ethyl Di-p-tolylphosphinite.

This experiment was run in the same way as described in Example 2, but a larger amount of all of the reactants was employed, except for the catalyst, $Tolyl_2POEt$. In this case, 30 mg of catalyst was used with 6 g of hexamethylbenzene, 6.7 ml of isopropanol, 7 mL of cyclohexane, 60 ml of toluene and 20 ml of acrylonitrile. A sample of this solution was sealed in an NMR tube under argon and heated together with the pressure tube at 60°C. At intervals the pressure tube was removed from the oil bath and sampled in the

usual way. At the same time, the NMR tube was removed. Analysis by $^{31}P$ NMR showed all of the phosphorus in the pressure tub had been converte to P(V) after the dimerization has proceeded for 127 hours, due to repeated openings of the tube, whereas most of the phosphorus in the NMR tube had remained in the form of P(III). The results are summarized in Table II.

Example 4

Long-Term Stability of Phopshinited Polystyrene.

Attempts to run the dimerization in an H-reactor using the polymeric catalyst, ethyl p-methoxyphenyphosphinited polystyrene; filtering off the reaction solution, drying the polymer, and reusing it resulted in a rapid decline in the performance of the catalysts from one run to the next. This may be due to a number of factors, especially concentration of the dimers and oligomers in the presence of the catalyst. To avoid this problem, the dimerization was run in the same way as described in Example 2 with the soluble catalyst. Thus, 400 g of phosphinited polystyrene was combined in a solvent reservoir with 6 g of hexamethylbenzene, 6.7 ml of isopropanol, 7 ml of cyclohexane, 60 ml of toluene and 20 ml of acrylonitrile, heated at 60°C and sampled at intervals. At the end of the experiment, the polymer was recovered and analyzed by $^{31}P$ NMR spectroscopy: all of the phosphorus had been converted to P(V) apparently due to repeated openings of the tube. The results are summarized in Table III.

TABLE I

Dimerization of Acrylonitrile to 1,4-Dicyano-1-butene[a]

| Catalyst | Time (h) | % Conv. | % Sel.[f] |
|---|---|---|---|
| (p-Tolyl)$_2$PO—i-Pr[b] | 3 | 28 | 85 |
| | 21 | 85 | 75 |
| Ph$_2$POEt[b] | 3 | 9[e] | 80[e] |
| | 26 | 49 | 81 |
| | 72 | 81 | 76 |
| (p-Tolyl)$_2$POEt[b] | 3 | 36 | 96 |
| | 24 | 89[e] | 91 |
| (p-Tolyl)$_2$POEt[b] | 3 | 25 | 88 |
| | 24 | 87[e] | 99[e] |
| 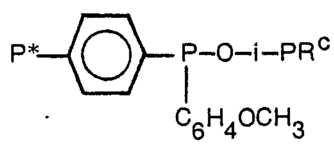 | 3 | 23 | 68 |
| | 24 | 77 | 75 |
| 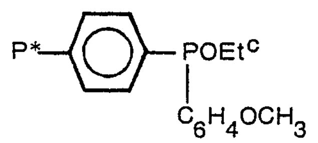 | 3 | 24 | 80 |
| | 24 | 80 | 76 |
| 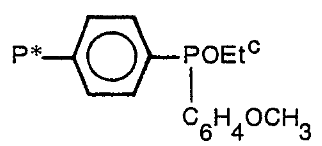 | 3 | 17[e] | 80[e] |
| | 24 | 68 | 88 |
| 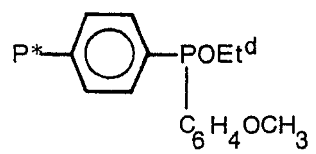 | 3 | 46 | 89 |
| | 6 | 65 | 88 |
| | 12 | 82 | 85 |
| 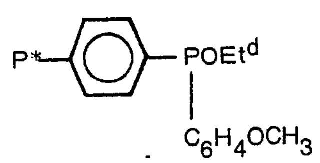 | 3 | 58 | 98 |

Footnotes to Table I

[a]Conditions: 800 mg of hexamethylbenzene, 1 ml of cyclohexane, 3 ml of acrylonitrile, 9 mL of toluene, 1 ml of i-PrOH, T=60°C, P=1.5 atm

[b]100 µl

[c]200 mg

[d]500 mg

[e]Results at low or high conversion are approximate due to uncertainty in integration of the G.C. peaks.

[f]% selectivity represents the yield of cis- and trans-1,4-dicyano-1-butene based on acrylonitrile consumed.

*Polystyrene cross-linked with 1 wt.% divinylbenzene.

TABLE II
Stability of Ethyl Di-p-Tolylphosphinite[a]

| Time (h) | % Conv. | % Selec.[b] | % P(III) |
|---|---|---|---|
| 0 | — | — | 83 |
| 13 | 35 | 94 | 82 |
| 21 | 48 | 97 | 82 |
| 35 | 64 | 98 | 79 |
| 43 | 67 | 91 | — |
| 57 | 72 | 92 | 76 |
| 127 | 68 | 92 | 63 |

[a]Conditions: 0.24 mmol of catalyst, 6.0 g of hexamethylbenzene, 7 ml of cyclohexane, 20 ml of acrylonitrile, 6.7 ml of isopropanol, 60 ml of toluene, 60°C in a sealed NMR tube.
[b]Selectivity represents the yield of cis- and trans-1,4-dicyano-1-butene based on acrylonitrile consumed.

TABLE III
Stability of Phosphinited Polystyrene[a]

| Time (h) | % Conv. | % Selec.[b] |
|---|---|---|
| 19 | 20 | 67 |
| 26 | 26 | 62 |
| 44 | 36 | 62 |
| 67 | 43 | 58 |
| 91 | 47 | 53 |

[a]Conditions: 400 mg catalyst:

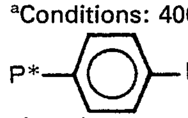 P–(p–$CH_3C_6H_4$–)–O–i–$C_2H_5$ wherein 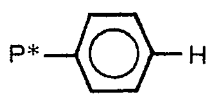

is polystyrene crosslinked with 1 weight % divinylbenzene, 6.0 g of hexamethylbenzene, 7 ml of cyclohexane, 20 ml of acrylonitrile, 6.7 ml of isopropanol, 60 ml of toluene, 60°C.
[b]Selectivity represents the yield of cis- and trans-1,4-dicyano-1-butene based on acrylonitrile consumed.

Example 5

Acrylonitrile Dimerization using Flow Reactor.

This Example was run in the apparatus illustrated in Figure 2. A flow reactor was assembled using two 0.9 × 30 cm glass, water-jacketed HPLC columns 13, 19, a piston-type HPLC pump 11 and a reservoir 2 fitted with stopcocks 4, 5 and a side arm 3 to allow addition of reagents with the exclusion of atmospheric oxygen and moisture. The exit of the second column 19 was attached to a pressure gauge 23, a three-way stopcock 25 and a graduated receiver 27. The inlet tube 26 to the receiver 27 was attached via a side arm to a mercury bubbler (not shown) which allowed application of 40 kPa (30 cm Hg) pressure to the system. At least 27 kPa (20 cm Hg) pressure is necessary to prevent bubble formation in the reactor column 19 and drying columns 13 and 16.

The two columns were fitted with adjustable plungers and stopcocks at each end. A three-way stopcock 17 was used at the bottom (inlet) end of the column 19 holding the catalyst resin. The two

columns were dried at 150°C, then placed while still hot in the port of a dry box. In the dry box, one column 13 was charged with 10 g of 16 mm pellets of 3A molecular sieves which had been activated at 200°C while being evacuated on a vacuum line, then allowed to cool under dry argon. The second column 19 was charged with 2.0 g (2.9 mmol of phosphorus) of the polystyrene catalyst bearing ethyl p-methoxyphenylphosphinite previously described in Example 4. Both columns were closed and removed from the dry box, then attached to the remainder of the system. Column 16 was not used in this experiment. The solvent reservoir was filled, under argon, with a degassed and dry solvent mixture of diisopropyl phenylphosphonite (20 µl), toluene (50 ml) and isopropanol (5 ml).

The solvent mixture was pumped through the columns at a rate of 22 ml/h until all bubbles of argon had been removed, then the water circulating bath was turned on, the system pressurized to 40 kPa (30 cm Hg) and the temperature of the columns 13, 19 raised to 60°C. The catalyst resin had swollen to about 13 ml volume.

In a specially designed reagent vessel 1, was placed 150 µl diisopropyl phenylphosphonite. The flask was attached to a vacuum line and the following dry reagents distilled in: toluene (270 ml), isopropanol (27 ml), cyclohexane (20.6 g, 26 ml) and acrylonitrile (67.8 g, 84 ml).

Vessel 1 was filled with argon, warmed to room temperature and the reagents thoroughly mixed. Vessel 1 was then attached to reservoir 2 via the sidearm 8 which was repeatedly evacuated and filled with argon. The reservoir 2 was evacuated and the contents of flask B transferred to it by opening the stopcock 9 above the filter 10. Pumping speed was adjusted to 7.8 ml/h and this point was designated as time 0 h.

The effluent from the reactor was collected in 125 ml graduated receivers 27 which were changed periodically at intervals of 10—13 h. Evaporation of each of these samples at 35°C (133 Pa or 1 mm Hg) left an oil which consisted of the acrylonitrile dimers and higher oligomers. The samples were further analyzed by gas chromatography and by Kugelrohr distillation of the dimers [linear and branched at 80—100°C (13.3 Pa or 0.1 mm Hg)]. Percent selectivities to dimers (see Table IV) were 90—91% a mixture of dimers (92% linear vs 8% branched dimers).

At intervals, samples of the reaction mixture were taken using the three-way stopcocks 17 and 25 before and after the catalyst bed. Gas chromatographic analysis of the amount of acrylonitrile present relative to cyclohexane allowed calculation of the percent conversion. The percent conversion slowly declined during the experiment from 63% initially to 40% at 184 hours (see Figure 2).

Additional batches of the reagent mixture were prepared and transferred to the reservoir when needed. Percent conversions were measured for periods when the flow rate was 7.7 ± 0.1 ml/h.

At 184 hours the last reservoir of reagents had been consumed and a mixture of toluene:isopropanol (10:1) was added to purge all of the remaining reagents through the system. The combined collected fractions contained 109.64 g of products, equivalent to 2.066 moles of acrylonitrile converted. This represents 714 catalyst turnovers. Results are summarized in Table IV and Figure 3.

After about 30 hours of reaction, the pump had to be disconnected and a valve was changed. After replacement of the valve and reconnection of the pump, the reaction was continued but the percent conversion started to decline faster. It is believed that this interruption caused contamination of the catalyst by moisture and oxygen. It is further believed that if this interruption had not occurred and if the reagent mixture had been more effectively dried such as by use of polystyrene-bound dialkyl phosphonite (e.g., placed in column 16 as shown in Figure 2), the decline in the activity of the catalyst as measured by the percent conversion would have been lower.

TABLE IV
Acrylonitrile Dimerization Products

| Receiver | Vol. ml | Wt g | (residue)[a] Wt. g | (dimers)[b] Wt. g | % Selec.[c,d] to dimers |
|---|---|---|---|---|---|
| 1 | 103 | 88.9 | 9.76 | 8.87 | 91 |
| 2 | 98.5 | 86.2 | 10.91 | | |
| 3 | 84.5 | 73.2 | 9.14 | | |
| 4 | 84 | 72.5 | 8.09 | 7.33 | 91 |
| 5 | 80 | 68.7 | 7.11 | | |
| 6 | 73 | 62.8 | 6.62 | | |
| 7 | 77.5 | 67.5 | 7.01 | 6.31 | 90 |
| 8 | 80 | 68.6 | 6.86 | | |
| 9 | 98 | 84.7 | 7.79 | | |
| 10 | 80 | 67.5 | 6.01 | 5.45 | 91 |
| 11 | 91 | 78.2 | 6.82 | | |
| 12 | 73 | 62.6 | 5.32 | | |
| 13 | 94 | 80.3 | 5.22 | 5.57 | 90 |
| 14 | 94 | 80.4 | 5.97 | | |
| 15 | 120 | | 4.92 | | |
| Combined samples | | | 1.09 | | |
| Total | | | 109.64 | | |

[a]After evaporation of reagents at 35°C (133 Pa or 1 mm Hg)
[b]Isolated by Kugelrohr distillation at 80—100°C (13.3 Pa or 0.1 mm Hg).
[c]92% 1,4-dicyano-1-butenes, 8% methyleneglutaronitrile.
[d]% Selectivity to DCB—1 = % Selec. to dimers × 92% (% DCB—1 in dimers).

Example 6

Preparation of the polystyrene-bound phosphonous dichloride Ⓟ—$C_6H_4$—$PCl_2$

In a typical preparation, 1 g (9.6 mmol) polystyrene resin cross-linked with 1 weight % divinylbenzene (Bio Beads. S—X1, 200—400 mesh) and 1.33 g (100 mmol) anhydrous aluminum chloride ($AlCl_3$) were placed under an inert atmosphere into one side of an H-reactor, as described in Example 1, and equipped with a reflux condenser with an argon inlet; 12.5 ml (excess) of degassed $PCl_3$ were syringed, under a stream of argon, into the reactor which was then placed in an oil bath at 60°C and stirred for 3 hours. The reaction mixture was degassed and filtered through the coarse glass frit and the filtrate poured off under argon. The polymer product was washed extensively with dry THF to remove residual $AlCl_3$ and dried *in vacuo*; yield: 1.7 g, $^{31}P$ NMR (swollen in $CH_2Cl_2$) δ 162 ppm (phosphonous dichloride). Phosphorus, 13.0% by weight, was incorporated into polymer indicating that ca. 75% of the rings were functionalized.

Example 7

Preparation of the polystyrene-bound diisopropyl phosphonite.

One gram (4.9 mmol) of the phosphonous dichloride was charged under an inert atmosphere into an H-reactor which was then evacuated on the vacuum line. Fifteen ml of dry pyridine were distilled onto the polymer and 1.2 ml (147 mmol) of dry isopropanol were distilled into the opposite leg of the reactor. The polymer suspension was stirred at 0°C for 1 hour while the isopropanol slowly distilled over. The mixture was warmed to room temperature and stirred an additional hour. The reaction was filtered through a glass

frit, and the the filtrate was poured off under argon. The polymer was washed repeatedly with dry methylene chloride and dried *in vacuo*. The $^{31}P$ NMR spectrum contained a peak at 162 ppm (broad peak) indicative of phosphonite phosphorus.

Example 8

This example illustrates the use of polystyrene-bound diisopropylphosphonite of Example 7 as a drying agent in acrylonitrile dimerization using the flow reactor of Example 5. A water-jacketed HPLC column 16 is placed in line 15 after column 13 and before the catalyst column 19 of Figure 2. The procedure of Example 5 is followed. A lower decline in the % conversion after a longer reaction time is expected due to a more complete removal of water.

Example 9

Preparation of polystyrene-bound 3,4,5-trimethylphenyl phosphinous chloride.

One gram (4.9 mmol) of the polystyrene-bound phosphonous dichloride and 0.72 g (5.4 mmol) of $AlCl_3$ were charged under an inert atmosphere into an H-reactor equipped with a reflux condenser with an argon inlet. Fifteen ml (excess) of 1,2,3-trimethylbenzene were syringed in under a stream of argon and suspension was stirred at 60°C for 6 hours. The filtrate was poured off under argon, and the polymer was washed with dry THF, then with THF/pyridine in the ratio of 15/2 and dried *in vacuo*. The $^{31}P$ NMR spectrum contained a peak at 85 ppm (phosphinous chloride phosphorus) and a smaller peak due to dichloride at 162 ppm. In another experiment, the H-reactor was charged as described above and the suspension was stirred under a stream of argon at 100°C for 1 hour. The polymer was washed and dried as described above. The $^{31}P$ NMR spectrum indicated that ca. 95% of the phosphorus was in the form of phosphinous chloride (peak at 85 ppm) and that only *ca.* 5% of unreacted starting material was present (peak at 162 ppm).

Example 10

Preparation of the polystyrene-bound isopropyl 3,4,5-trimethylphenyl phosphinite.

One gram of the phosphinous chloride prepared as described in Example 8 was charged into an H-reactor under an inert atmosphere and evacuated on the vacuum line. 15 ml of dry THF and 0.5 ml of dry pyridine were distilled onto the polymer and 0.5 ml (6.1 mmol) of dry isopropanol was distilled into the opposite leg. The reaction conditions of Example 7 were followed. The polymer was washed extensively with dry THF. The $^{31}P$ NMR spectrum contained a peak at 107 ppm indicative of phosphinite phosphorus (*ca.* 90% of P) and a smaller peak at 44 ppm (*ca.* 10% of P) due to an unidentified impurity. See Figure 1b.

Example 11a

Reaction 3,4,5-$(CH_3)_3C_6H_2PCl_2$ and Polystyrene at 60°C

One gram (9.6 mmol) of polystyrene resin crosslinked with 1% divinylbenzene (Bio Beads, S—X1, 200—400 mesh) and 1.33 g (10.0 mmol) $AlCl_3$ were charged under an inert atmosphere into an H-reactor equipped with a reflux condenser and argon inlet. The polymer was swollen in 10 ml of dry 1,1,2,2-tetrachloroethane which was added (syringe) under argon. 3,4,5-$(CH_3)_3C_6H_2PCl_2$ (2.8 g, 12.7 mmol) dissolved in 2 ml dry 1,1,2,2-tetrachloroethane was added to reaction mixture via syringe under argon and the reaction mixture so formed was heated at 60°C for 4 hours. The suspension was degassed and filtered through the glass frit, and the filtrate poured off under argon. The polymer was washed extensively with dry THF. The $^{31}P$ NMR spectrum showed no incorporation of phosphorus into the polymer.

Example 11b

Reaction of 3,4,5-$(CH_3)_3$—$C_6H_2PCl_2$ with Polystyrene at 100°C

The reaction described in Example 11a was repeated excepting that the reaction mixture so formed was heated at 100°C for 2 hours. The dark brown reaction mixture produced was filtered under argon and a red-brown solid recovered and washed three times with 15 ml portions of THF. The yellow-orange solid was washed with dry $CH_2Cl_2$, again with THF (the color of solid remained unchanged) and dried *in vacuo* to give a gummy yellow-orange solid. The $^{31}P$ NMR spectrum of the gummy yellow-orange solid (swollen in $CH_2Cl_2$) contained a large broad multiplet of unassigned peaks at 70 ppm, 51.4 ppm, 46 ppm and 36 ppm, said unassigned peaks indicative of incorporation of phosphorus into polymer and a small peak at 162 ppm indicative of a phosphonous dichloride. There was no peak at 85 ppm indicative of uncomplexed polymer-bound phosphinous chloride.

Example 11c

Attempted Preparation of Polystyrene-bound isopropyl 3,4,5-trimethylphenylphosphinite

The reaction product of Example 11b was treated in accordance with the apparatus and procedure of Example 10. Dry THF and pyridine were distilled onto *ca.* 700 mg of the gummy yellow-orange solid of Example 11b and removed by filtration. Fresh dry THF (12 ml) dry pyridine (2 ml) and dry isopropanol (2.0 ml) were distilled onto polymer. The reaction conditions and apparatus of Example 7 were then used excepting that the reaction product was washed with THF and a THF-isopropanol mixture and dried *in vacuo*. There was recovered 0.53 g of a yellow-orange gummy solid. The $^{31}P$ NMR spectrum contained a broad multiplet of peaks centered around 30 ppm indicative of phosphorus (V). The phosphorus signal was

very weak, indicating little phosphorus was incorporated and the spectrum contained no peak around 110 ppm indicating no phosphinite phosphorus was present.

Example 12

Attempted Dimerization of ACN using Trimethylsilyl Dibutylphosphinite

A dimerization reaction mixture was prepared in accordance with the apparatus and procedure of Example 2 from cyclohexane (1 ml), isopropanol (1 ml), acrylonitrile (3 ml) toluene (9 ml), hexamethylbenzene (1.334 g) and trimethylsilyl dibutylphosphonite (0.1 ml) which was prepared by the procedure described by M. Grayson et al. in *Tetrahedron*, 1967, Vol. 23, at page 1085 et seq. The solution was heated at 60°C for 18 h, then sampled under argon. GC analysis showed no reaction products and no conversion of the acrylonitrile. Analysis of the reaction mixture by $^{31}P$ NMR showed one major peak at δ 46 ppm, corresponding to a rearrangement product, and nothing at 117 ppm relative to 85% $H_3PO_4$ where the starting phosphinite phosphorus appears.

Example 13

4-(2-Hydroxypropyl)polystyrene

A 3.0 g sample of brominated polystyrene containing 29% bromine and crosslinked with 1 wt% of divinylbenzene was placed on one side of an H-reactor. Butyl lithium (20 ml, 2.4 M) was syringed into the other side, the hexane solvent was mostly evaporated and replaced by 30 ml dry toluene. The toluene solution was filtered onto the polymer and the mixture was heated at 60°C for 3 hours. The polymer was filtered, the toluene poured off and fresh toluene was distilled in. After several washings the toluene was poured off and the polymer dried. Fresh toluene (30 ml) was then distilled onto the polymer followed by 1.1 ml of propylene oxide (15.7 mmol). The mixture was stirred at room temperature for 20 hours, then filtered, the solvent poured off and 20 ml of isopropanol were distilled in. After stirring, the polymer was filtered again and then washed in the air with isopropanol (2 × 100 ml), isopropanol containing several drops of concentrated hydrochloric acid (2 × 100 ml) and again with isopropanol (2 × 100 ml). Drying overnight in a vacuum oven at 60°C gave 2.5 g of product. Analysis for hydroxyl content gave a value of 3.7 meq OH/g of polymer.

Examples 14—15

Partially Phosphinited Hydroxypropylpolystyrene

A 1.0 g sample of the hydroxypropylpolystyrene prepared as described in Example 13 was treated in an H-reactor with di-p-tolylphosphinous chloride (0.37 g, 1.49 mmol) in pyridine (15 ml). After 3 days the mixture was filtered, washed with pyridine once and with dichloromethane several times. The dried polymer weighed 0.95 g. Phosphorus NMR Spectrum contained only a single broad peak at *ca.* δ 107 ppm. Fully phosphinited hydroxypropylpolystyrene was prepared similarly using an excess of phosphinous chloride. The $^{31}P$ NMR spectrum contained only a single broad peak at *ca.* δ 107 ppm.

Example 16

Partial Phosphination of TDI-Cross-linked PVAL

A sample of polyvinyl alcohol cross-linked with about 5% tolylenediisocyanate was dried *in vacuo* at 60°C. The polymer (0.33 g) was placed in an H-reactor and treated with diphenylphosphinous chloride (0.50 g, 2.25 mmol) in pyridine (15 ml) for 4 days at room temperature. After filtration, the polymer was washed several times with dichloromethane and dried. Yield: 0.23 g. Phosphorus NMR showed a substantial amount of phosphorus incorporation, with about 90% in the phosphinite form. The sample was recovered from the NMR tube, dried and used in a dimerization run (See Example 21) but no conversion of ACN was observed. (See Table V).

Example 17

Complete Phosphination of TDI-Cross-linked PVAL

A 0.5 g sample of dried, TDI-cross-linked, 80% hydrolyzed polyvinyl acetate was treated with a slight excess of di-p-tolylphosphinous chloride (2.0 g, 8 mmol) in pyridine (15 ml) for 4 days at room temperature. The polymer was filtered, washed with dichloromethane and dried. Yield: 0.49 g. The phosphorus NMR spectrum showed a strong peak in the region (δ 110 ppm) expected for phosphinite and a smaller peak in the P(V) region. Thus, although there was no weight gain, the recovered material contained an appreciable amount of phosphorus. This polymer was used in a dimerization reaction with neopentyl alcohol. (See Example 21). No conversion of ACN was indicated by GLC.

Example 18

Fully Phosphinited TOYOPEARL®

A 1.0 g of dried, coarse TOYOPEARL® was treated with excess di-p-tolylphosphinous chloride (2.5 g, 10 mmol) in pyridine (20 ml) in an H-reactor. After 2 days at room temperature the reaction was stopped. The mixture could not be filtered, so the pyridine was pumped off and dichloromethane distilled in. The polymer was stirred briefly, then filtered. After several more washings with dichloromethane, the product was dried on the vacuum line. An appreciable amount of material was lost due to the fine particles being

pulled up into the vacuum line. Yield: 1.45 g. The $^{31}P$ NMR spectrum showed a strong broad peak at δ 115 ppm phosphinite phosphorus and a number of sharp resonances of lower intensities due to monomeric phosphorus species.

Example 19

Partially Phosphinited TOYOPEARL® (30%)

Superfine TOYOPEARL® (3.0 g) was treated with about 0.3 equivalents of di-p-tolylphosphinous chloride (1.4 g) in pyridine for 2 days at room temperature. The product was filtered in a glove bag and washed with dichloromethane, then dried overnight in a vacuum oven. It was then washed repeatedly with dichloromethane in an H-reactor and redried. Yield: 2.7 g. Phosphorus NMR shows a broad peak in the phosphinite region and a smaller broad peak in the P(V) region.

Example 20

Partially Phosphinited TOYOPEARL® (6%)

A 4.0 g sample of coarse TOYOPEARL® was treated for about 0.06 equivalents of di-p-tolylphosphinous chloride (0.44 g, 1.77 mmol) in pyridine (30 ml). The mixture was stirred overnight at room temperature, filtered under argon and washed with dichloromethane, then dried on the vacuum line. Yield: 4.35 g. The phosphorus NMR showed a single broad resonance at δ 115 ppm (due to phosphinite phosphorus) and a small amount of a monomeric P(V) compound.

Comparative Example 21

Dimerizations of acrylonitrile using hydroxyl-containing polymer-bound phosphinites of Examples 14—20 were run in accordance with the procedure of Example 2 excepting that no isopropanol was added. In runs 6—7 wherein all the OH groups of polymer-bound catalyst were converted into phosphinite at the start of the reaction; neopentyl alcohol was added. See Table V for a summary of results.

ACN Dimerization Runs 6—8 of Example 22

Dimerization using Fully Phosphinited Toyopearl and Neopentyl Alcohol

A dimerization reaction was carried out in accordance with the procedure and apparatus of Example 2 using the fully phosphinited TOYOPEARL of Example 18 as the catalyst and neopentyl alcohol as the added alcohol. After 20 hours, the reaction mixture was analyzed. Conversion was 90% and selectivity to 1,4-dicyano-1-butenes also about 90%. The reaction mixture was filtered, concentrated and the phosphorus NMR Spectrum was obtained on a methylene chloride solution of the residue. The $^{31}P$ NMR spectrum showed several peaks, the major ones being at δ 112 ppm, due to neopentyl di-p-tolylphosphinite, and at δ 29 ppm, probably due to the tertiary phosphine oxide. The $^{31}P$ NMR spectrum of the recovered polymer showed no polymer-bound phosphorus. Repetition of the experiment gave 14% conversion after 3 hours, with >90% selectivity; in addition to the peaks noted above, the $^{31}P$ NMR showed a smaller peak at δ −50 ppm due to the pentacoordinate phosphorane.

TABLE V

ACN Dimerization Runs Using Various Polymer-Bound Phosphinite Without Added Alcohol[a]

| | Polymer[b] | %P[c] | Time (h) | Results |
|---|---|---|---|---|
| 1 | PS—OP—Tolyl[1/2] | 40 | 22.5 | No ACN conversion |
| 2 | PS—OP—Tolyl[1/2] | 40 | 5 | Mainly P(V) |
| 3 | PS—OP—Tolyl[1/2] | 5 | 24 | No ACN conversion |
| 4 | TDI—PVAL[3] | 40 | 6 | No ACN conversion |
| 5 | TDI—PVA[4] | 100 | 24 | No ACN conversion |
| 6 | TOYOPEARL®[5] | 100[d] | 20 | 90% conversion;<br>90% selectivity to DCB—1;<br>No polymer-bound phosphorus after 20 h;<br>neopentyl di-p-tolylphosphinite present |
| 7 | TOYOPEARL®[5] | 100[d] | 3 | 14% conversion;<br>90 selectivity to DCB—1;<br>No polymer-bound phosphorus after 3 h;<br>neopentyl di-p-tolyl-phosphonite and P(V) present |
| 8 | TOYOPEARL®[6] | 6 | 24 | 11% conversion;<br>37% selectivity to DCB—1 |

Footnotes to Table V

[a] 60°C. Typical amounts: 9 ml of toluene, 3 ml of ACN, 1 mL of cyclohexane, 0.2 mmol of phosphorus.
[b] PS—O = 4-(2′-hydroxy-1′-propylpolystyrene crosslinked with 10% of divinyl benzene (See Examples 14—15); TDI—PVAL = 5% Tolylene diisocyanate cross-linked polyvinyl alcohol.
[c] Percent of polymer-bound OH groups derivatized with P-(p-$CH_3C_6H_5)_2$ groups.
[d] Neopentyl alcohol was added.

Preparation of Polymers listed in Table V
[1] See Examples 14, 15.
[2] See Examples 14, 15.
[3] See Example 16.
[4] See Example 17.
[5] See Example 18.
[6] See Example 20.

**Claims**

1. A polymer-bound alkyl diarylphosphinite catalyst which is substantially free of C=O, OH, NH or SH groups and is of the formula (I):

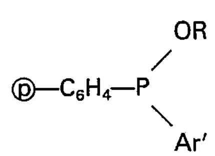

(I)

wherein Ⓟ—$C_6H_4$— is bonded to the trivalent phosphorus atom through a P—C bond and is a pendant aryl group of a polymer matrix derived from a polymer Ⓟ—$C_6H_5$ containing aryl groups, at least 1% of the pendant aryl groups of the polymer matrix being bound to phosphorus, Ar′ is an aromatic group of the formula:

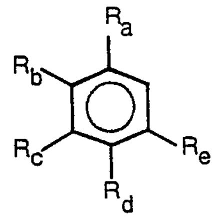

wherein $R_a$, $R_b$, $R_c$, $R_d$ and $R_e$ are independently heteroatom-containing electron-donating groups selected from —$OR^3$ and —$N(R^4, R^5)$, or hydrogen, alkyl of 1 to 10 carbon atoms or cycloalkyl of 5 to 10 carbon atoms, or two of said $R_a$ to $R_e$ groups from part of a fused alicyclic ring and the remainder of said $R_a$ to $R_e$ groups are as hereinbefore defined, and R, $R^3$, $R^4$ and $R^5$ are independently straight or branched chain alkyl of 1 to 10 carbons or cycloalkyl of 5 to 10 carbon atoms.

2. A catalyst according to claim 1 where at least one of $R_a$ to $R_e$ is a said electron-donating group.

3. A catalyst according to claim 1 or 2 wherein the polymer is polystyrene cross-linked with 1—40 weight% divinylbenzene.

4. A catalyst according to claim 1, 2 or 3 wherein the polymer is in the form of micro- or macroeticular beads.

5. A catalyst according to claim 1 of the formula: ⓟ—$C_6H_4$—P(p-$CH_3O$—$C_6H_4$)OR wherein ⓟ—$C_6H_4$ is derived from a polymer which is polystyrene and wherein R is ethyl or isopropyl.

6. A catalyst according to claim 1 of the formula: ⓟ—$C_6H_4$—P[$(CH_3)_3C_6H_2$]OR wherein ⓟ—$C_6H_4$— is derived from a polymer which is polystyrene and wherein R is cyclohexyl, methyl, ethyl or isopropyl.

7. A process for preparation of a catalyst as claimed in claim 1 which comprises the steps of:

(a) brominating at least 1% of the pendant aromatic groups of a polymer matrix of the formula ⓟ—$C_6H_5$;

(b) contacting the brominated aromatic-containing polymer produced in step (a) with at least a stoichiometric amount of a lithium compound selected from $LiNH_2$, $LiNHR^7$, $LiNR^7R^8$ and $LiR^7$, wherein $R^7$ and $R^8$ are independently alkyl of 1 to 8 carbons or cycloalkyl of 5 to 12 carbons;

(c) contacting the lithiated aromatic-containing polymer produced in step (b) with an aryl phosphorus compound selected from Ar'P(X)OR, $Ar'P(OR)_2$ and $Ar'PX_2$ wherein Ar' and R are as defined in claim 1 and X is halogen;

(d) recovering a polymeric substance having the formula ⓟ—$C_6H_4$—P(Ar')OR or ⓟ—$C_6H_4$—P(Ar')X when $Ar'P(X)_2$ is used in step (c); and

(e) when ⓟ—$C_6H_4$—P(Ar')X is formed in step (d) converting it into ⓟ—$C_6H_4$—P(Ar')OR by reaction with ROH and pyridine.

8. A process according to claim 7 wherein in step (c) Ar'P(X)OR in the form of p-$CH_3OC_6H_4P(Cl)OR$ is used and wherein R is cyclohexyl, methyl, ethyl or a 2-alkyl group having 3 to 10 carbon atoms.

9. A process according to claim 7 or 8, which further comprises treating the polymeric substance recovered to produce a polymeric substance substantially free of lithium cations and bases.

10. A process for the preparation of a catalyst as claimed in claim 1 which comprises the steps of:

(a) contacting a polymer ⓟ—$C_6H_5$ with $PX_3$, in the presence of an effective amount of a Friedel-Crafts catalyst, for a time sufficient to produce a polymer wherein at least 1% of the pendant aromatic groups are of the formula —$C_6H_4$—$PX_2$ wherein X is F, Cl, Br or I;

(b) contacting the polymer produced in step (a) in the presence of an effective amount of a Friedel-Crafts catalyst, with an aromatic compound Ar'H, wherein Ar' is as defined in claim 1, for a time sufficient to produce a polymer having the formula ⓟ—$C_6H_4$—P(Ar')X; and

(c) contacting the product of step (b) with an alkanol or cycloalkanol of the formula ROH, wherein R is as defined in claim 1, in the presence of a base for a time sufficient to produce a polymer of formula ⓟ—$C_6H_4$—P(Ar')OR.

11. A process according to claim 10 wherein in step (b), Ar'H is a trimethylbenzene and wherein in step (c) methanol, ethanol, cyclohexanol or a 2-alkanol having 3 to 10 carbon atoms is used and the base is pyridine.

12. A process according to claim 10 or 11 which further comprises treating the polymer produced in step (b) with a polar solvent or with a polar solvent in the presence of a base to produce a polymer substantially free of residual Friedel-Crafts catalyst.

13. A substance having the formula ⓟ—$C_6H_4$—P[$(CH_3)_3C_6H_2$—]—Cl.

14. A process for the conversion of acrylonitrile into 1,4-dicyano-1-butene which comprises contacting a liquid phase comprising acrylonitrile with an effective amount of a polymer-bound alkyl diarylphosphinite catalyst as claimed in any one of claims 1 to 5 for a time sufficient to effect conversion to a stream containing 1,4-dicyano-1-butene.

15. A process according to claim 14 wherein the contacting is performed in a flow reactor, under substantially anhydrous and oxygen-free conditions and in the substantial absence of bases and lithium cations.

16. A process according to claim 15 wherein the catalyst is ⓟ—$C_6H_4$—P[$(CH_3)_3C_6H_2$—]OR.

17. A process according to claim 15 wherein the catalyst is Ⓟ—$C_6H_4$—P(p-$CH_3OC_6H_4$—)OR.

18. A process according to claim 15, 16 or 17 wherein the liquid acrylonitrile phase further comprises an inert solvent and a proton-donating solvent and wherein the total water content of the liquid phase is less than 50 ppm and wherein the liquid phase, prior to contacting it with the catalyst, is treated with an effective amount of a drying agent comprising a polymer-bound di(cyclo)alkyl arylphosphonite for a time sufficient to reduce the total water content of the liquid phase to a value less than 15 ppm.

19. A process according to claim 18 which further comprises recovering the at least partly deactivated drying agent comprising polymer-bound alkyl phosphinate units having the formula Ⓟ—$C_6H_4$—P=O(OR)(H) produced by the interaction of at least some of the polymer-bound dialkyl arylphosphonite units with water in the liquid phase, contacting said deactivated drying agent with an effective amount of $PCl_3$ for a time sufficient to produce polymer-bound alkyl arylphosphonous dichloride units and contacting said polymer-bound alkyl arylphosphonous dichloride units with a primary or secondary alkanol of 1—10 carbons in the presence of base for a time sufficient to produce a polymer-bound dialkyl arylphosphonite suitable for further use as a drying agent.

20. A process according to claim 18 wherein the drying agent is polystyrene-bound diisopropyl arylphosphonite, the inert solvent is toluene and the proton-donating solvent is isopropanol.

**Patentansprüche**

1. Polymergebundener Alkyldiarylphosphinit-Katalysator, der im wesentlichen frei von C=O—, OH—, NH— oder SH— Gruppen ist und die Formel (I) hat:

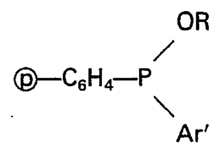

(I)

worin Ⓟ—$C_6H_4$— an das dreiwertige Phosphoratom über eine P—C-Bindung gebunden ist und eine an einer Polymermatrix, die sich von einem Arylgruppen enthaltenden Polymer Ⓟ—$C_6H_5$ herleitet, hängende Arylgruppen ist, wobei wenigstens 1% der an der Polymermatrix hängenden Arylgruppen an Phosphor gebunden ist, Ar' eine aromatische Gruppe der Formel

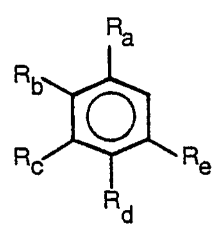

bedeutet, worin $R_a$, $R_b$, $R_c$, $R_d$ und $R_e$ unabhängig voneinander ein Heteroatom enthaltende Elektronendonorgruppen sind, die aus —$OR^3$ und —N($R^4$, $R^5$) oder Wasserstoff, Alkyl mit 1 bis 10 Kohlenstoffatomen oder cycloalkyl mit 5 bis 10 Kohlenstoffatomen ausgewählt sind, oder zwei der Gruppen $R_a$ bis $R_e$ einen Teil eines verschmolzenen alicyclischen Ringes bilden und der Rest der Gruppen $R_a$ bis $R_e$ wie oben definiert ist und R, $R^3$, $R^4$ und $R^5$ unabhängig voneinander geradkettige oder verzweigtkettige Alkylgruppen mit 1 bis 10 Kohlenstoffatomen oder cycloalkylgruppen mit 5 bis 10 Kohlenstoffatomen sind.

2. Katalysator nach Anspruch 1, bei dem wenigstens eine der Gruppen $R_a$ bis $R_e$ eine solche Elektronendonorgruppe ist.

3. Katalysator nach Anspruch 1 oder 2, bei dem das Polymer mit 1 bis 40 Gewichts-% Divinylbenzol vernetztes Polystyrol ist.

4. Katalysator nach Anspruch 1, 2 oder 3, bei dem das Polymer in der Form von Mikro- oder Makroetikularperlen vorliegt.

5. Katalysator nach Anspruch 1 der Formel: Ⓟ—$C_6H_4$—P(p-$CH_3O$—$C_6H_4$)OR, worin Ⓟ—$C_6H_4$ sich von einem Polymer herleitet, das Polystyrol ist, und worin R Ethyl oder Isopropyl ist.

6. Katalysator nach Anspruch 1 der Formel: Ⓟ—$C_6H_4$—P[$(CH_3)_3C_6H_2$]OR, worin Ⓟ—$C_6H_4$— sich von einem Polymer herleitet, das Polystyrol ist, und worin R Cyclohexyl, Methyl, Ethyl oder Isopropyl ist.

7. Verfahren zur Herstellung eines Katalysators nach Anspruch 1 mit den Stufen, bei denen man:

(a) wenigstens 1% der an einer Polymermatrix der Formel Ⓟ—$C_6H_5$ hängenden aromatischen Gruppen bromiert,

(b) das in Stufe (a) erzeugte bromierte aromatische Gruppen enthaltende Polymer mit wenigstens einer stöchiometrischen Menge einer Lithiumverbindung, die unter $LiNH_2$, $LiNHR^7$, $LiNR^7R^8$ und $LiR^7$ ausgewählt ist, behandelt, worin $R^7$ und $R^8$ unabhängig voneinander Alkylgruppen mit 1 bis 8 Kohlenstoffatomen oder Cycloalkylgruppen mit 5 bis 12 Kohlenstoffatomen sind,

(c) das in Stufe (b) erzeugte Polymer, das mit Lithium behandelte aromatische Gruppen enthält, mit einer Arylphosphorverbindung behandelt, die aus Ar'P(X)OR, $Ar'P(OR)_2$ und $Ar'PX_2$ ausgewählt ist, worin Ar' und R wie in Anspruch 1 definiert sind und X Halogen bedeutet,

(d) eine Polymersubstanz mit der Formel ⓟ—$C_6H_4$—P(Ar')OR oder ⓟ—$C_6H_4$—P(Ar')X gewinnt, wenn $Ar'P(X)_2$ in Stufe (c) verwendet wird, und

(e) wenn in Stufe (d) ⓟ—$C_6H_4$—P(Ar')X gebildet wird, dieses durch Umsetzung mit ROH und Pyridin in ⓟ—$C_6H_4$—P(Ar')OR umwandelt.

8. Verfahren nach Anspruch 7, worin in Stufe (c) Ar'P(X)OR in der Form von $p\text{-}CH_3OC_6H_4P(Cl)OR$ verwendet wird und worin R Cyclohexyl, Methyl, Ethyl oder eine 2-Alkylgruppe mit 3 bis 10 Kohlenstoffatomen ist, ist.

9. Verfahren nach Anspruch 7 oder 8, welches weiterhin eine Behandlung der gewonnen Polymersubstanz unter Bildung einer von Lithiumkationen und Basen im wesentlichen freien Polymersubstanz einschließt.

10. Verfahren zur Herstellung eines Katalysators nach Anspruch 1 mit den Stufen, bei denen man:

(a) ein Polymer ⓟ—$C_6H_5$ mit $PX_3$ in Gegenwart einer wirksamen Menge eines Friedel-Crafts-Katalysators während ausreichender Zeit behandelt, um ein Polymer zu bilden, worin wenigstens 1% der daran hängenden aromatischen Gruppen die Formel-$C_6H_4PX_2$ hat, worin X F, Cl, Br oder I ist,

(b) das in Stufe (a) gebildete Polymer in Gegenwart einer wirksamen Menge eines Friedel-Crafts-Katalysators mit einer aromatischen Verbindung Ar'H, worin Ar' wie in Anspruch 1 definiert ist, ausreichend lange behandelt, um ein Polymer der Formel ⓟ—$C_6H_4$—P(Ar')X zu erzeugen, und

(c) das Produkt der Stufe (b) mit einem Alkanol oder Cycloalkanol der Formel ROH, worin R wie in Anspruch 1 definiert ist, in Gegenwart einer Base ausreichend lange behandelt, um ein Polymer der Formel ⓟ—$C_6H_4$—P(Ar')OR zu erzeugen.

11. Verfahren nach Anspruch 10, bei dem in Stufe (b) Ar'H ein Trimethylbenzol ist und bei dem in Stufe (c) Methanol, Ethanol, Cyclohexanol oder ein 2-Alkanol mit 3 bis 10 Kohlenstoffatomen verwendet wird und die Base Pyridin ist.

12. Verfahren nach Anspruch 10 oder 11, das weiterhin eine Behandlung des in Stufe (b) erzeugten Polymers mit einem polaren Lösungsmittel oder mit einem polaren Lösungsmittel in Gegenwart einer Base einschließt, um ein von restlichem Friedel-Crafts-Katalysator im wesentlichen freies Polymer zu erzeugen.

13. Verbindung der Formel ⓟ—$C_6H_4$—$P[(CH_3)_3C_6H_2$—]—Cl.

14. Verfahren zur Umwandlung von Acrylnitril in 1,4-Dicyano-1-buten, bei dem man eine Acrylnitril enthaltende flüssige Phase mit einer wirksamen Menge eines polymergebundenen Alkyldiarylphosphinit-Katalysators gemäß einem der Ansprüche 1 bis 5 ausreichende lange behandelt, um eine Umwandlung in einen 1,4-Dicyano-1-buten enthaltenden Strom zu bewirken.

15. Verfahren nach Anspruch 14, bei dem die Behandlung in einem Fließreaktor unter im wesentlichen wasserfreien und sauerstofffreien Bedingungen und im wesentlichen in Abwesenheit von Basen und Lithiumkationen erfolgt.

16. Verfahren nach Anspruch 15, bei dem der Katalysator ⓟ—$C_6H_4$—$P[(CH_3)_3C_6H_2$—]OR ist.

17. Verfahren nach Anspruch 15, bei dem der Katalysator ⓟ—$C_6H_4$—$P(p\text{-}CH_3OC_6H_4$—)OR ist.

18. Verfahren nach Anspruch 15, 16 oder 17, bei dem die flüssige Acrylnitrilphase außerdem ein inertes Lösungsmittel und ein Protonendonor-Lösungsmittel enthält und worin der gesamte Wassergehalt der flüssigen Phase geringer als 50 ppm ist und worin die flüssige Phase vor ihrer Behandlung mit dem Katalysator mit einer wirksamen Menge eines Trocknungsmittels, das polymergebundene Di-(cyclo)-alkyl-arylphosphinit umfaßt, ausreichend lange behandelt wird, um den Gesamtwassergehalt der flüssigen Phase auf einen Wert geringer als 15 ppm zu vermindern.

19. Verfahren nach Anspruch 18, bei dem weiterhin das wenigstens teilweise deaktivierte Trocknungsmittel gewonnen wird, das polymergebundene Alkylphosphinateinheiten der Formel ⓟ—$C_6H_4$—P=O(OR)(H) aufweist, die durch Wechselwirking wenigstens einiger der polymer gebundene Dialkylarylphosphiniteinheiten mit Wasser in der flüssigen Phase erzeugt wurden, dieses deaktivierte Trocknungsmittel mit einer wirksamen Menge $PCL_3$ ausreichend lange behandelt, um polymergebundene Alkylarylphosphordichlorideinheiten zu erzeugen, und die polymergebundenen Alkylarylphosphordichlorideinheiten mit einem primären oder sekundären Alkohol mit 1 bis 10 Kohlenstoffatomen in Gegenwart einer Base ausreichend lange behandelt, um ein für weitere Verwendung als Trocknungsmittel geeignetes polymergebundenes Dialkylarylphosphonit zu erzeugen.

20. Verfahren nach Anspruch 18, bei dem das Trocknungsmittel polystyrolgebundenes Diisopropylarylphosphonit ist, das inerte Lösungsmittel Toluol ist und das Protonendonor-Lösungsmittel Isopropanol ist.

**Revendications**

1. Un catalyseur alkyldiarylphosphinite lié à un polymère, qui est sensiblement dépourvu de groupes C=O, OH, NH ou SH et qui a la formule (I):

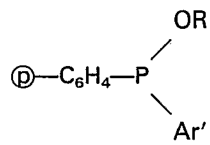

(I)

dans laquelle la formule ⓟ—$C_6H_4$— est liée à l'atome de phosphore trivalent par l'intermédiaire d'une liaison P—C et est un groupe aryle pendant d'une matrice polymère dérivée d'un polymère ⓟ—$C_6H_5$ contenant des groupes aryle, au moins 1% des groupes aryle pendants de la matrice polymère étant liés au phosphore, Ar' est un groupe aromatique de formule:

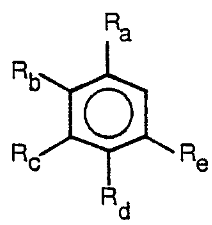

dans laquelle $R_a$, $R_b$, $R_c$, $R_d$ et $R_e$ sont indépendamment des groupes contenant des hétéroatomes, donneurs d'électrons, choisis parmi —$OR^3$ et —$N(R^4, R^5)$, ou l'hydrogène, un alkyle de 1 à 10 atomes de carbone ou cycloalkyle de 5 à 10 atomes de carbone ou deux desdits groupes $R_a$ à $R_e$ font partie d'une combinaison alicyclique condensée et le reste desdits groupes $R_a$ à $R_e$ sont tels que définis ci-dessus, et R, $R^3$, $R^4$ et $R^5$ sont indépendamment un alkyle à chaîne droite ou ramifié de 1 à 10 carbones ou un cycloalkyle de 5 à 10 atomes de carbone.

2. Un catalyseur selon la revendication 1 dans lequel au moins l'un de $R_a$ à $R_e$ est un dit groupe donneur d'électrons.

3. Un catalyseur selon la revendication 1 ou 2 dans lequel le polymère est du polystyrène réticulé avec 1—40% en poids de divinylbenzène.

4. Un catalyseur selon la revendication 1, 2 ou 3 dans lequel le polymère est sous forme de billes micro ou macro réticulaires.

5. Un catalyseur selon la revendication 1 de la formule: ⓟ—$C_6H_4$—P(p-$CH_3O$—$C_6H_4$)OR dans laquelle ⓟ—$C_6H_4$ est dérivé d'un polymère qui est le polystyrène et dans laquelle R est un éthyle ou un isopropyle.

6. Un catalyseur selon la revendication 1 de la formule: ⓟ—$C_6H_4$—P[$(CH_3)_3C_6H_2$]OR dans laquelle ⓟ—$C_6H_4$ est dérivé d'un polymère qui est le polystyrène et dans laquelle R est un cyclohexyle, un méthyle, un éthyle ou un isopropyle.

7. Un procédé de préparation d'un catalyseur selon la revendication 1 qui comprend les étapes suivantes:

(a) bromer au moins 1% des groupes aromatiques pendants d'une matrice polymère de formule ⓟ—$C_6H_5$;

(b) mettre en contact le polymère contenant les groupes aromatiques bromés préparé à l'étape (a) avec au moins une quantité stoechiométrique d'un composé du lithium choisi parmi $LiNH_2$, $LiNHR^7$, $LiNR^7R^8$ et $LiR^7$, dans lesquels $R^7$ et $R^8$ sont indépendamment un alkyle de 1 à 8 carbones ou un cycloalkyle de 5 à 12 carbones,

(c) mettre en contact le polymère contenant les groupes aromatiques lithié préparé à l'étape (b) avec un composé d'arylphosphore choisi parmi Ar'P(X)OR, Ar'$P(OR)_2$ et Ar'$PX_2$ dans lesquelles Ar' et R sont tels que définis à la revendication 1 et X est un halogène;

(d) récupérer une substance polymère ayant la formule ⓟ—$C_6H_4$—P(Ar')Or ou ⓟ—$C_6H_4$—P(Ar')X lorsque l'on emploie Ar'$P(X)_2$ à l'étape (c); et

(e) lorsque ⓟ—$C_6H_4$—P(Ar')X est formé à l'étape (d) le convertir en ⓟ—$C_6H_4$—P(Ar')OR par réaction avec ROH et de la pyridine.

8. Un procédé selon la revendication 7 dans lequel on se sert à l'étape (c) d'Ar'P(X)OR sous forme de p-$CH_3OC_6H_4P(Cl)OR$ et dans laquelle R est un cyclohexyle, un méthyle, un éthyle ou un groupe alkyle-2 ayant de 3 à 10 atomes de carbone.

9. Un procédé selon la revendication 7 ou 8, qui comprend en outre le traitement de la substance polymère récupérée pour produire une substance polymère sensiblement dépourvue de cations lithium et de bases.

10. Un procédé de préparation d'un catalyseur selon la revendication 1 qui comprend les étapes suivantes:

(a) mettre en contact un polymère ⓟ—$C_6H_5$ avec $PX_3$, en présence d'une quantité efficace d'un catalyseur de Friedel-Crafts, pendant un temps suffisant pour produire un polymère dans lequel au moins 1% des groupes aromatiques pendants ont la formule —$C_6H_4$—$PX_2$ dans laquelle X est F, Cl, Br ou I;

(b) mettre en contact le polymère obtenu à l'étape (a) en présence d'une quantité efficace d'un catalyseur de Friedel-Crafts, avec un composé aromatique Ar'H, dans lequel Ar' est tel que défini à la revendication 1, pendant un temps suffisant pour obtenir un polymère ayant la formule ⓟ—$C_6H_4$—P(Ar')X; et

(c) mettre en contact le produit de l'étape (b) avec un alcanol ou un cycloalcanol de formule ROH, dans laquelle R est tel que défini à la revendication 1, en présence d'une base pendant un temps suffisant pour produire un polymère de formule ⓟ—$C_6H_4$—P(Ar')OR.

11. Un procédé selon la revendication 10 dans lequel à l'étape (b), Ar'H est un triméthylbenzène et dans lequel à l'étape (c), on se sert de méthanol, d'éthanol, de cyclohexanol ou d'un alcanol-2 ayant de 3 à 10 atomes de carbone et la base est la pyridine.

12. Un procédé selon la revendication 10 ou 11 qui comprend en outre le traitement du polymère produit à l'étape (b) avec un solvant polaire ou avec un solvant polaire en présence d'une base pour obtenir un polymère sensiblement dépourvu d'un catalyseur de Friedel-Krafts résiduel.

13. Une substance ayant la formule ⓟ—$C_6H_4$—P[$(CH_3)_3C_6H_2$—]—Cl.

14. Un procédé de transformation de l'acrylonitrile en dicyano-1,4 butène-1 qui comprend la mise en contact d'une phase liquide comprenant de l'acrylonitrile avec une quantité efficace d'un catalyseur alkyldiarylphosphinite lié à un polymère selon l'une quelconque des revendications 1 à 5, pendant un temps suffisant pour effectuer la transformation en un courant contenant du dicyano-1,4 butène-1.

15. Un procédé selon la revendication 14 dans lequel la mise en contact est effectuée dans un réacteur à écoulement, dans des conditions sensiblement anhydres et dépourvues d'oxygène, et sensiblement en absence de bases et de cations lithium.

16. Un procédé selon la revendication 15 dans lequel le catalyseur est ⓟ—$C_6H_4$—P[$(CH_3)_3C_6H_2$—]OR.

17. Un procédé selon la revendication 15 dans lequel le catalyseur est ⓟ—$C_6H_4$—P(p-$CH_3OC_6H_4$—)OR.

18. Un procédé selon la revendication 15, 16 ou 17 dans lequel la phase liquide d'acrylonitrile comprend en outre un solvant inerte et un solvant donneur de protons et dans lequel la teneur totale en eau de la phase liquide est inférieure à 50 ppm et dans lequel la phase liquide avant sa mise en contact avec le catalyseur, est traitée à l'aide d'une quantité efficace d'un agent déssechant comprenant un di(cyclo)alkyl-arylphosphonite lié à un polymère, pendant un temps suffisant pour réduire la teneur totale en eau de la phase liquide à une valeur inférieure à 15 ppm.

19. Un procédé selon la revendication 18 qui comprend en outre la récupération de l'agent déssechant au moins partiellement désactivé comprenant des motifs alkylphosphinate liés à un polymère, ayant la formule ⓟ—$C_6H_4$—P=O(OR)(H) obtenu par l'interaction d'au moins quelques uns des motifs dialkylarylphosphonite liés à un polymère avec de l'eau dans la phase liquide, la mise en contact dudit agent désséchant désactivé avec une quantité efficace de $PCl_3$ pendant un temps suffisant pour obtenir des motifs de dichlorure alkylarylphosphoneux liés à un polymère et la mise en contact desdits motifs de dichlorure alkylarylphosphoneux liés à un polymère avec un alcanol primaire ou secondaire de 1—10 carbones en présence de base, pendant un temps suffisant pour obtenir un dialkylarylphosphonite lié à un polymère convenable en vue d'un emploi ultérieur en tant qu'agent désséchant.

20. Un procédé selon la revendication 18 dans lequel l'agent désséchant est un diisopropylaryl-phosphonite lié à un polystyrène, le solvant inerte est du toluène et le solvant donneur de protons est l'isopropanol.

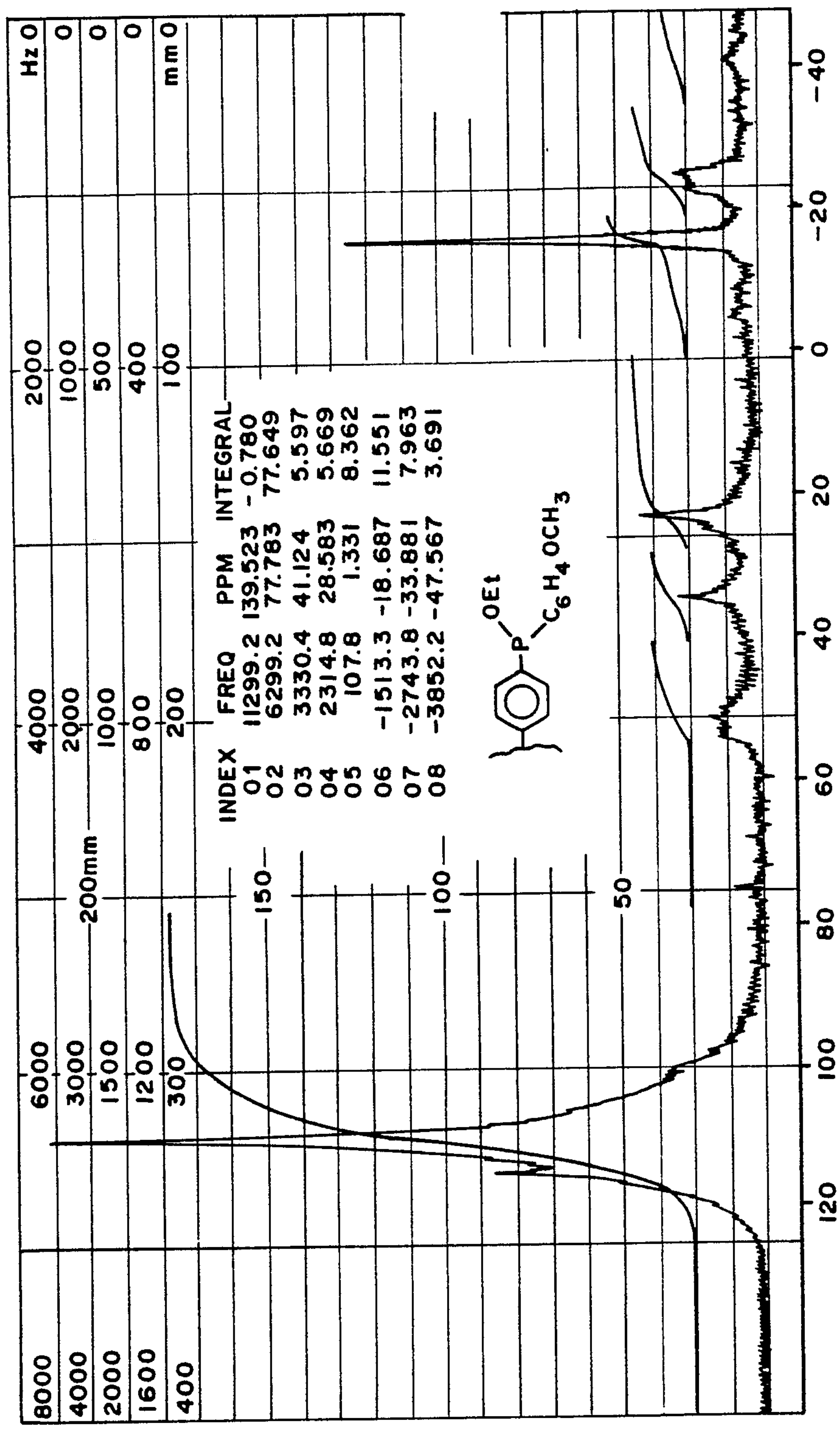


FIG. 1a $^{31}P$ NMR SPECTRUM OF PHOSPHINITED POLYSTYRENE IN DICHLOROMETHANE

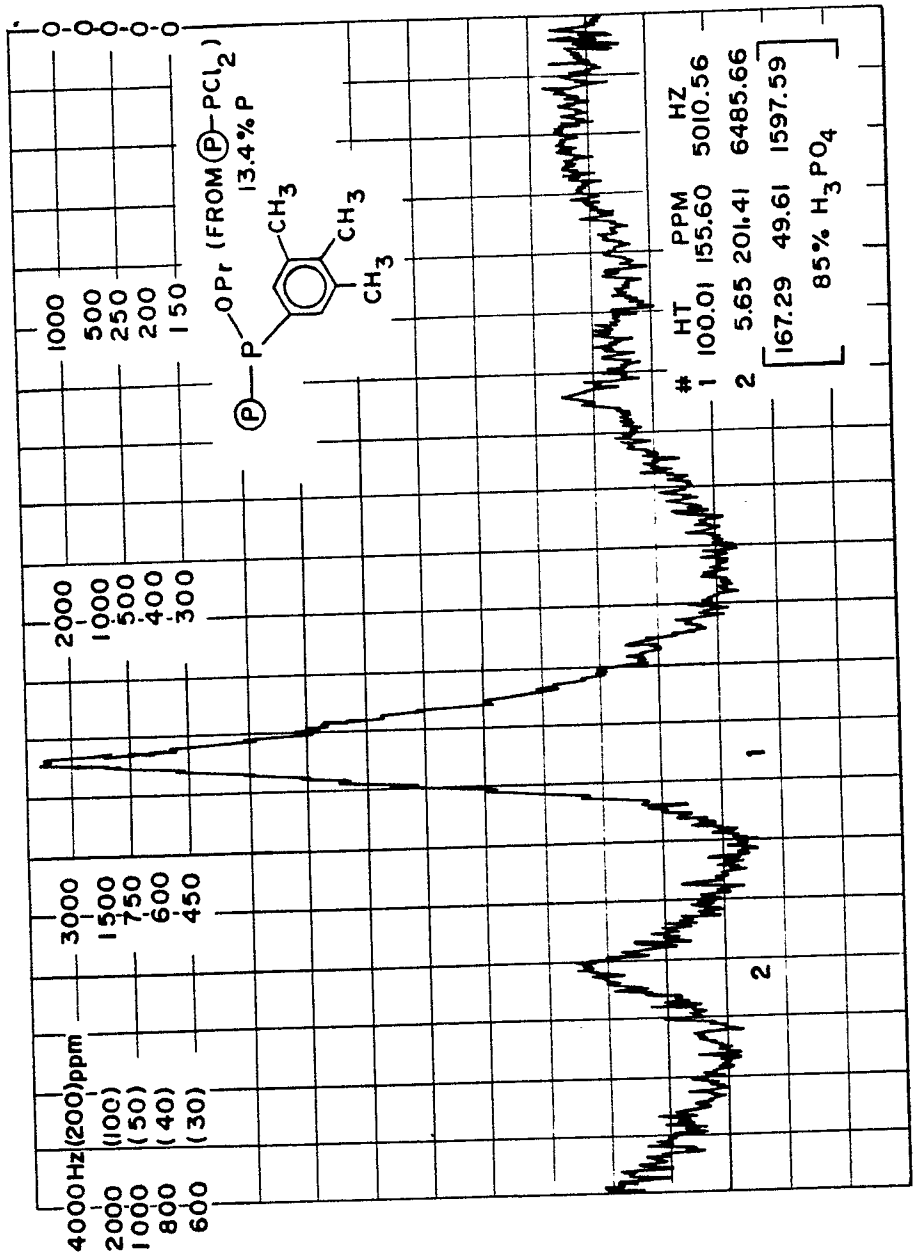
(FROM (P)-PCl2)
13.4% P
# HT PPM HZ
1 100.01 155.60 5010.56
2 5.65 201.41 6485.66
[167.29 49.61 1597.59]
85% H3PO4
4000Hz (200)ppm
2000 (100)
1000 (50)
800 (40)
600 (30)

FIG. 1b

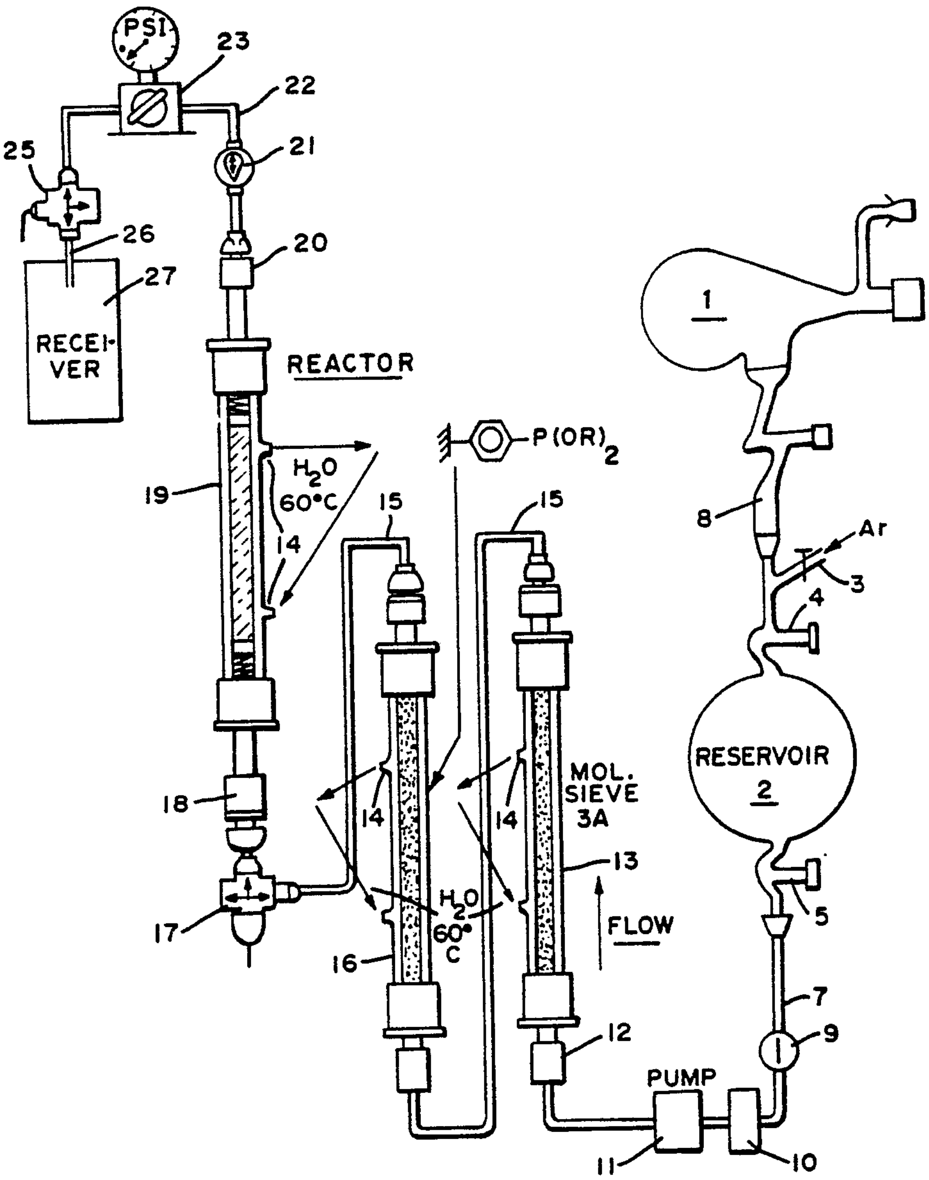
PSI
23
22
21
25
26
20
27
RECEI-
VER
REACTOR
1
H2O
60°C
P(OR)2
19
15
15
14
8
Ar
3
4
MOL.
SIEVE
3A
RESERVOIR
2
18
14
14
13
17
H2O
60°
C
FLOW
5
16
7
12
9
PUMP
11
10

FIG. 2

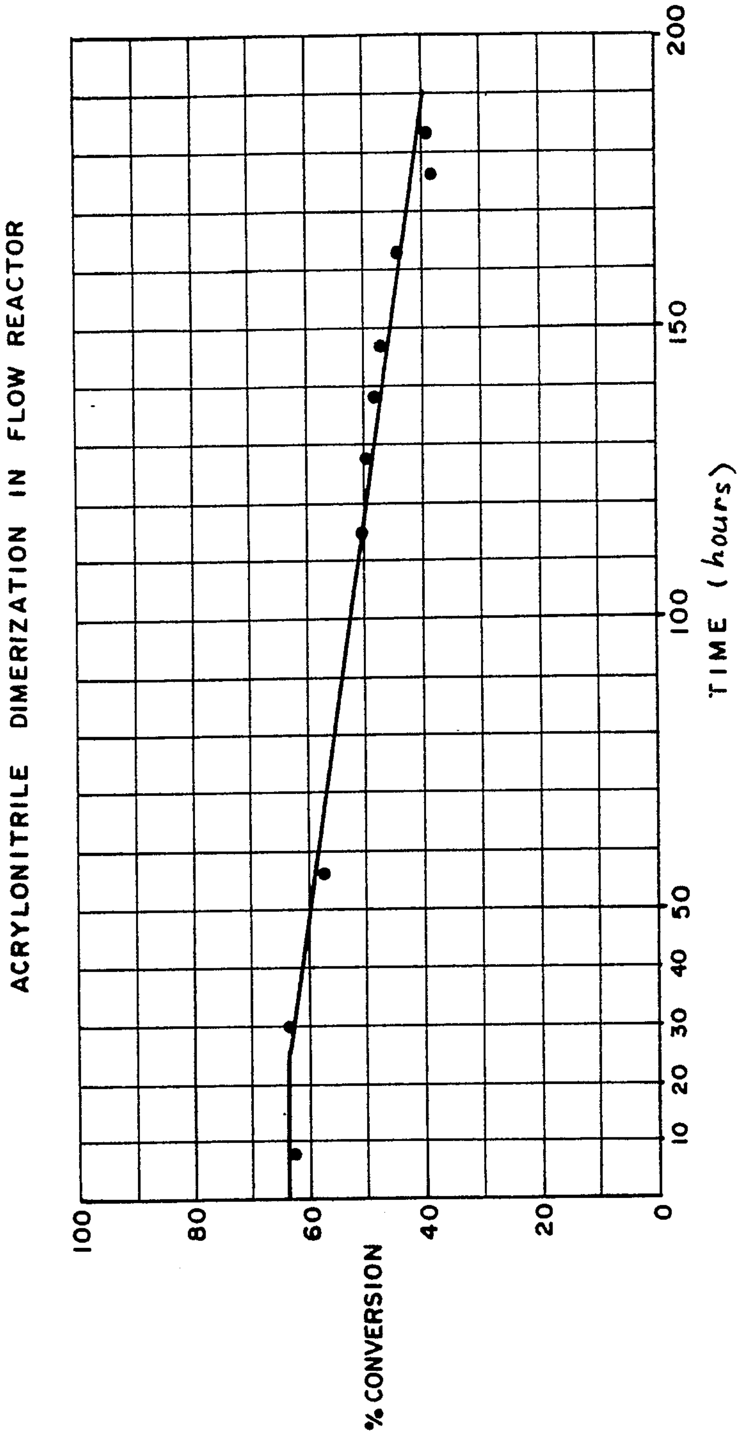
ACRYLONITRILE DIMERIZATION IN FLOW REACTOR
% CONVERSION
100
80
60
40
20
0
TIME (hours)
10
20
30
40
50
100
150
200

FIG. 3

FIG. 4

1)
2)
3)

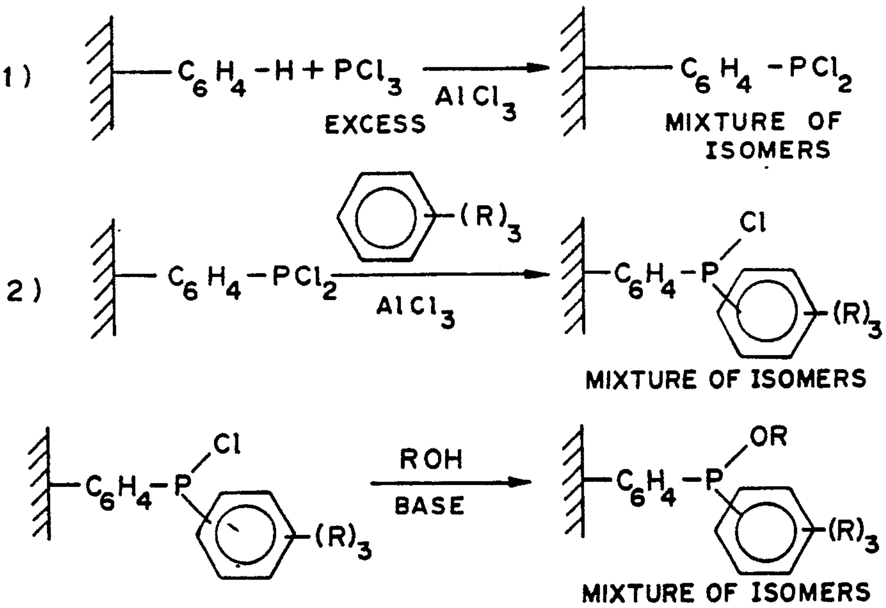


FIG. 5

1
2
3

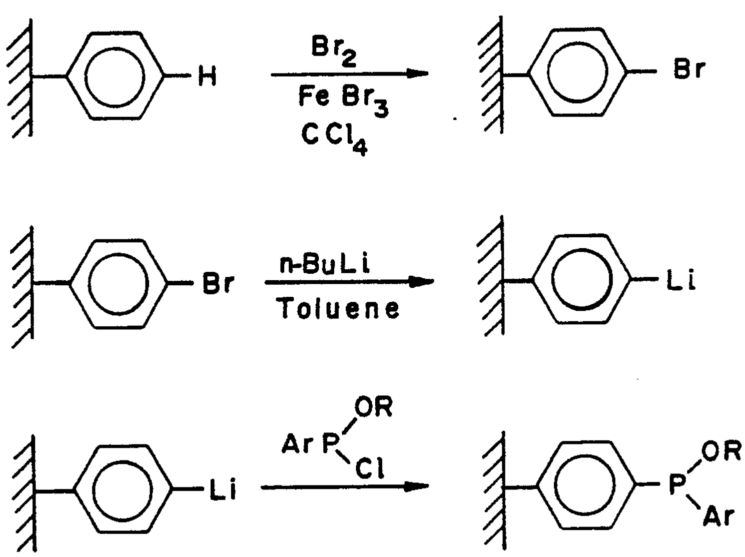

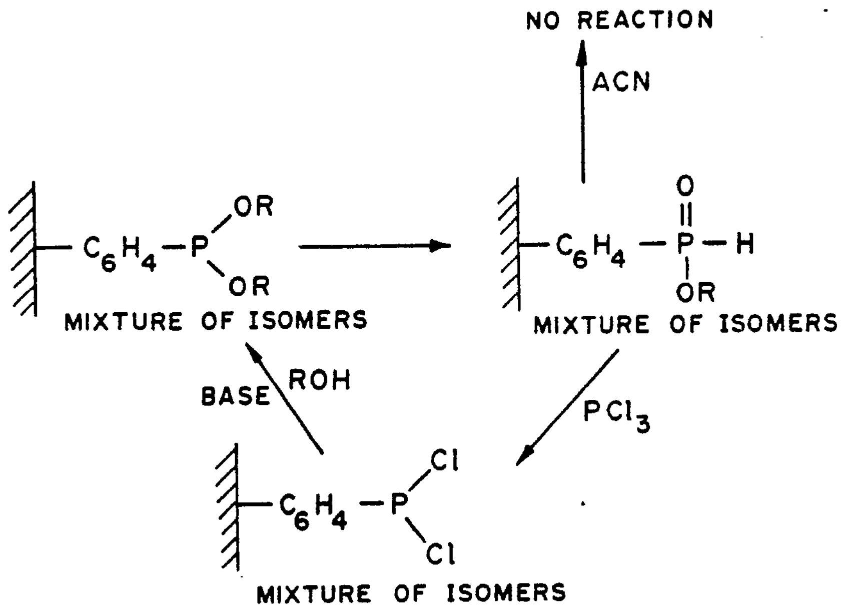
NO REACTION
ACN
MIXTURE OF ISOMERS
MIXTURE OF ISOMERS
BASE
ROH
PCl3
MIXTURE OF ISOMERS

FIG. 6

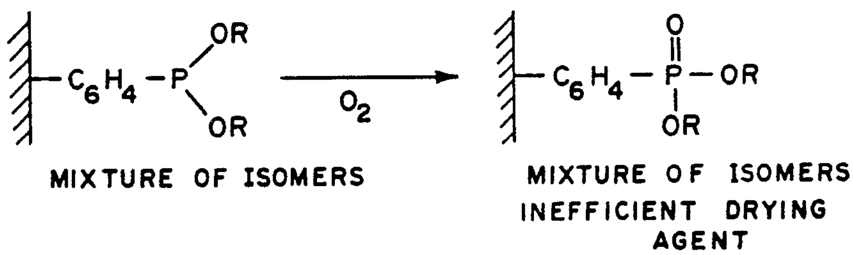
O2
MIXTURE OF ISOMERS
MIXTURE OF ISOMERS
INEFFICIENT DRYING AGENT

FIG. 7

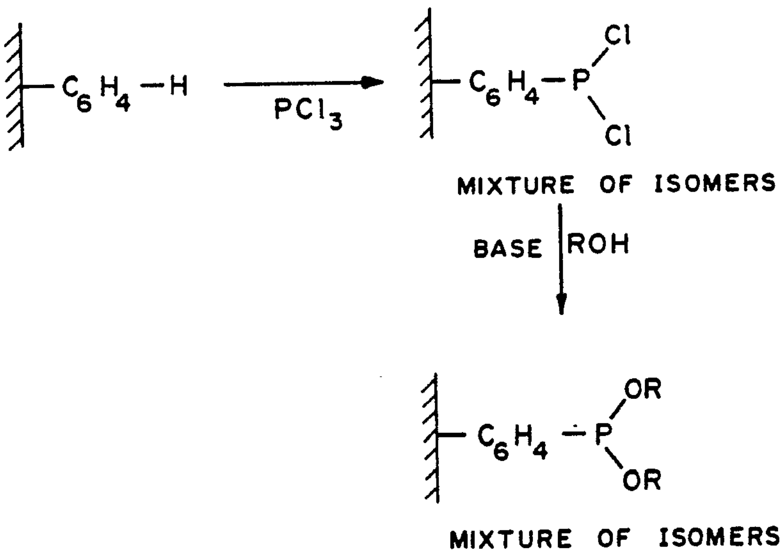


FIG. 8